# EUROPEAN PATENT SPECIFICATION

(11) **EP 1 131 338 B1**
(45) Date of publication and mention of the grant of the patent: **02.03.2005**
(21) Application number: 99970664.1
(22) Date of filing: 21.10.1999
(51) Int. Cl.: C07K 1/00, A61K 38/00, A61K 39/00, A61K 39/02, A61K 39/116, A61K 45/00, A61K 39/085, C07K 14/24

(54) **METHOD FOR THE PRODUCTION OF PURIFIED INVASIN PROTEIN AND USE THEREOF**
METHODE FÜR DIE HERSTELLUNG VON GEREINIGTEM INVASINPROTEIN UND SEINE ANWENDUNG
PROCEDE DE PRODUCTION DE PROTEINE INVASINE HAUTEMENT PURIFIEE ET UTILISATION DE CETTE PROTEINE

(30) Priority: 21.10.1998 US 105085 P; 01.06.1999 US 136754 P
(43) Date of publication of application: 12.09.2001
(73) Proprietor: THE UNIVERSITY OF KANSAS CENTER FOR RESEARCH, INC., Lawrence, KS 66044 (US); Walter Reed Army Institute of Research, Silver Spring, MY 20910-7500 (US)
(72) Inventor: PICKING, William, D., Lawrence, KS 66045 (US); PICKING, Wendy, L., Lawrence, KS 66045 (US); OAKS, Edwin, V., Gambrills, MD 21054 (US)
(74) Representative: Smaggasgale, Gillian Helen
(86) International application number: PCT/US1999/024931
(87) International publication number: WO 2000/023462

(56) References cited:
- CORTHESY ET AL.: 'A pathogen-specific epitope inserted into recombinant secretory immunoglobulin A is immunogenic by the oral route' THE JOURNAL OF BIOLOGICAL CHEMISTRY vol. 271, no. 52, 27 December 1996, pages 33670 - 33677, XP002923107
- PICKING ET AL.: 'Cloning, expression and affinity purification of recombinant Shigella flexneri invasion plasmid antigens IpaB and IpaC' PROTEIN EXPRESSION AND PURIFICATION vol. 8, no. 0117, 1996, pages 401 - 408, XP002923108
- MARQUART ET AL.: 'Soluble invasion plasmid antigen C (IpaC) from Shigella flexneri elicits epithelial cell responses related to pathogen invasion' INFECTION AND IMMUNITY vol. 64, no. 10, October 1996, pages 4182 - 4187, XP002923109

## Description

### FIELD OF THE INVENTION

This invention relates to compositions of highly purified recombinant invasin proteins and methods for the production of highly purified recombinant invasin proteins. Also provided in the present invention are adjuvant compositions comprising highly purified invasin proteins, use of invasin proteins in vaccine compositions, use of invasin proteins to stimulate an immune response, and the use of invasin proteins to deliver therapeutic or diagnostic materials to cells.

### BACKGROUND OF THE INVENTION

The enteroinvasive bacterial species of the genera *Shigella, Salmonella,* and enteroinvasive strains of *Escherichia coli* cause significant death and disease worldwide. *Shigella flexneri* is a gram negative enteric bacterium that is an important etiologic agent of bacillary dysentery. Shigellosis is a significant cause of infant mortality in underdeveloped regions of the world, where it also causes debilitating illness among travelers and personnel participating in humanitarian and peace-keeping ventures. *Shigella* (particularly S. *sonnei)* are also responsible for common-source outbreaks in developed nations when susceptible individuals are crowded together, such as in child daycare centers. Overt manifestations of shigellosis include fever, abdominal cramping, and loose scanty stools containing mucus and blood. These symptoms result from *S*. *flexneri* invasion of the colonic epithelium which is accompanied by the formation of localized lesions and severe inflammation. Similarly, enteroinvasive strains of *E*. *coli* contain an invasion plasmid which conveys a virulent *Shigella-*like phenotype upon this ordinarily benign member of the normal flora of the human gut.

*Salmonella spp.* are a well-known cause of food poisoning in the U.S. and worldwide. *Salmonella* are gram-negative enteric bacteria which widely occur in poultry and swine. Thus, the main methods of transmission of *Salmonella* to a human host include the ingestion of raw or undercooked egg and meat products, or foods which have been contaminated with bacteria from raw products. Symptoms of salmonellosis include nausea, fever, headache, abdominal cramps and diarrhea. These symptoms, like those of shigellosis, are caused by the invasion of the colonic epithelium by *Salmonella* which is accompanied by the formation of localized lesions and severe inflammation. S. *typhi* and paratyphoid bacteria cause typhoid fever, which has a 10% mortality rate among infected individuals. In contrast, less virulent species, such as *Salmonella enteritidis,* have a 1% mortality rate. There are an estimated two to four million cases of salmonellosis in the U.S. alone each year. The incidences of salmonellosis seem to be rising in U.S. and other industrialized nations, making salmonellosis a continuing significant health threat.

Entry of *S*. *flexneri, Salmonella typhimurium,* and enteroinvasive E. *coli* into epithelial cells has been called "pathogen-induced phagocytosis" and is characterized by localized actin polymerization at the inner face of the host cytoplasmic membrane at the site of bacterial contact. This leads to membrane ruffling and the formation of protrusions that surround and eventually engulf the bacterium. In *Shigella* infection, rapid lysis of the resulting phagocytic vacuole allows the bacterium to gain access to the target cell cytoplasm. In contrast, invasive *Salmonella* bacteria do not lyse the vacuole. A 31-kb fragment of the large virulence plasmid of S. *flexneri* encodes the components necessary for entry into the epithelial cell cytoplasm (Maurelli et al., *Infect. Immun.,* 49:164-171, 1985; Sasakawa et al., *J. Bacteriol.,* 170:2480-2484, 1988). Important regions within this fragment include: 1) the *mxi* and *spa* operons which encode components of a type III secretory apparatus that mediates secretion of the effectors of epithelial cell entry; and 2) the *ipa* operon which has eight open reading frames and encodes four immunogenic polypeptides that are involved in triggering pathogen entry into host cells. Proteins encoded by the *ipa* operon include invasion plasmid antigens (**Ipa**) A (70 kDa), B (62 kDa), C (42 kDa) and D (38 kDa) (Buysse et al., *J*. *Bacteriol.,* 169:2561-2569, 1987). The amino acid sequences of these proteins have been fully characterized (Venkatesan et al., *Proc. Natl. Acad. Sci. USA,* 85:9317-9321, 1988; Venkatesan et al., *Nuc. Acids Res.,* 18:1648, 1990). The invasins of other *Shigella* spp. are virtually identical to those of *S. flexneri* and are considered to be functionally interchangeable. The synthesis and secretion of IpaB, IpaC and IpaD are required for S. *flexneri* to invade the epithelial cells of the intestine.

The Ipa proteins are rapidly secreted from *S. flexneri* when the bacterium is incubated with epithelial cells. Following their secretion, IpaB and IpaC can be found as part of a protein complex that may also contain other bacterial proteins. The secreted IpaB/IpaC complex has been proposed to mediate pathogen entry. After binding, the Ipa complex may be responsible for eliciting signaling cascades that trigger changes in target cell protein kinase activities and it may promote lysis of the resulting phagosomal membrane.

The invasion protein antigen complex of *Shigella* is extremely immunogenic so that the vast majority of animals which have been exposed to a virulent *Shigella* strain develop antibodies to these proteins (Oaks et al., *Infect. Immun*., 53:57-63, 1986). The IpaC protein, in particular, has elicited much interest as an antigenic agent. Epitope mapping of the IpaC protein has revealed three clusters of particularly immunogenic amino acid sequences in the protein, between residues 1-61, 177-257, and 298-307 (Turbyfill et al., *Infect. Immun.,* 63:3927-3935, 1995). This observation ofIpaC's antigenic character has led to the suggestion that IpaC may be used as a "carrier" protein, in which a foreign epitope is recombinantly inserted in order to induce an immunogenic response to that epitope (Barzu, *Infect. Immun.,* 64:1190-1196, 1996). So far, such experiments have only produced IpaC in *Shigella* organisms, and have had to rely on these organisms to deliver IpaC to the target epithelial cells. Such efforts have shown disappointing immunological results.

The invasive strains of the *Salmonella* bacteria carry a chromosomal gene which encodes proteins with remarkable similarity to the invasins of *Shigella* (Kaniga et al., *J. Bacteriol*., 177:3965-3971, 1995; Kaniga et al., *J. Bacteriol.,* 177:7078-7085, 1995; see Table 1). These *Salmonella* invasin proteins (Sip's) are necessary for the bacterium to enter the epithelial cell, and are thought to function similarly to the Ipa's (Kaniga et al., *J*. *Bacteriol*., 177:3965-3971, 1995). The sequence of this set of invasins has also been fully disclosed (Kaniga et al., *J*. *Bacteriol.,* 177:3965-3971, 1995; Kaniga et al., *J. Bacteriol.,* 177:7078-7085, 1995).

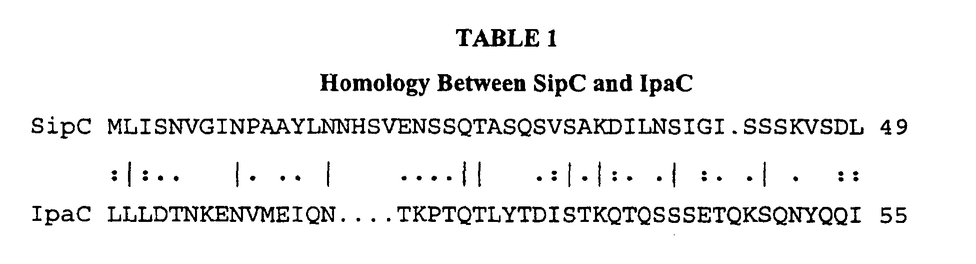

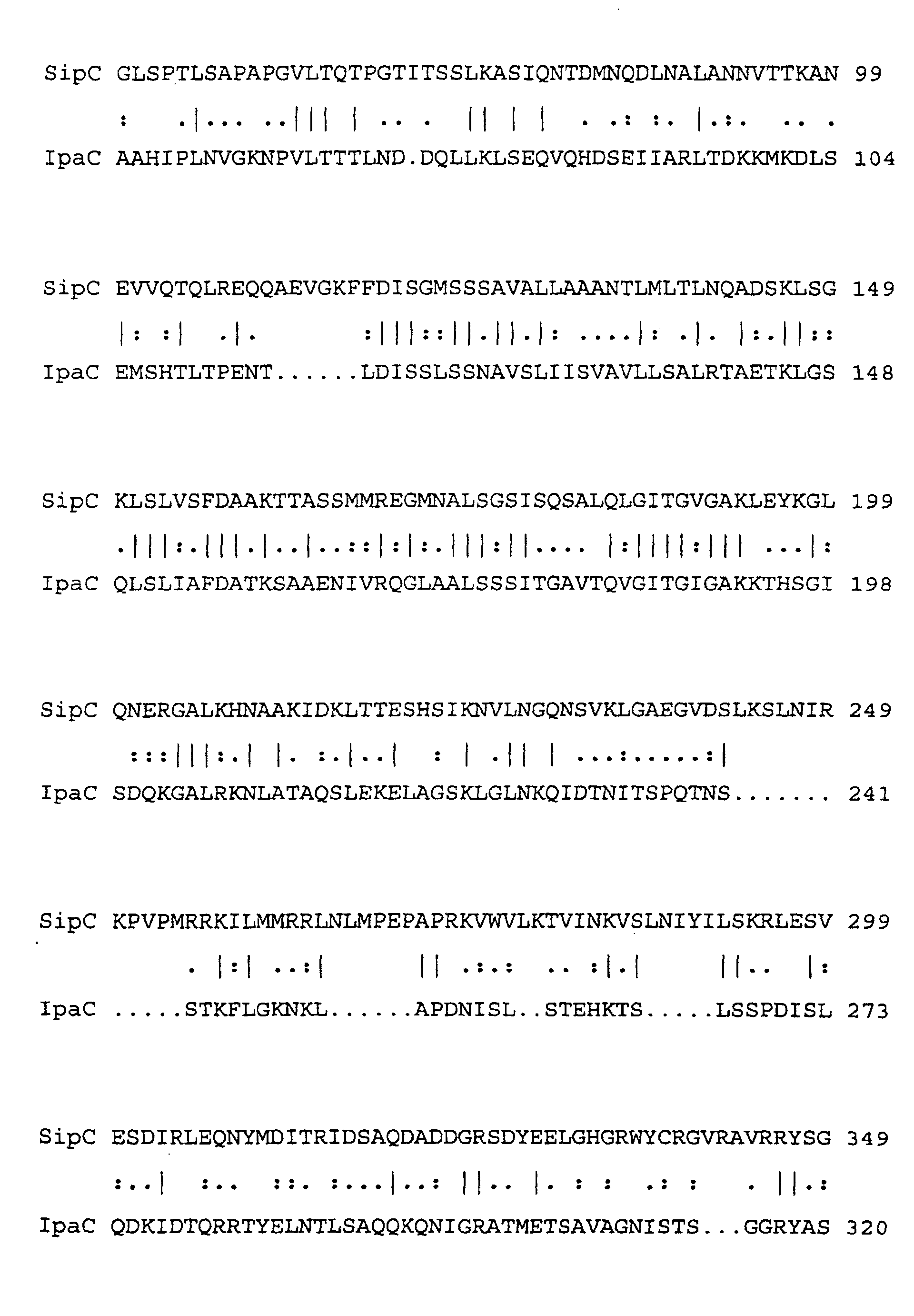

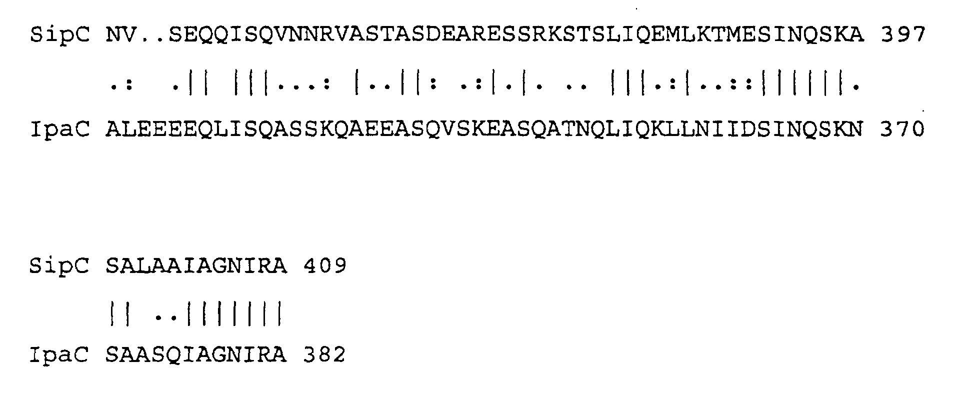

Sequence alignment of *Salmonella typhimurium* SipC and *Shigella flexneri* IpaC. Lines indicate complete identity; colons and periods indicate conservative amino acid substitutions. Here, IpaC has been numbered according to the Venkatesan starting amino acid.

Enteroinvasive *E. coli* also contains a plasmid which encodes proteins virtually identical to those contained on the *Shigella* invasion plasmid. In fact, enteroinvasive E. *coli* invasins have been shown to be cross-reactive with antibodies specific for *Shigella* invasin proteins. In addition, common genetic DNA probes have been used to identify both enteroinvasive *E. coli* and *Shigella spp.,* further demonstrating the homology of their invasion plasmids (U.S. Patents Nos. 4,816,389 and 5,041,372). The invasins of *E. coli* are generally considered to be identical to those of Shigella for all practical purposes, and are also often referred to as Ipa proteins.

The development of a vaccine for *Shigella* has long been a world health priority because of the significant health threat that this organism poses. Research has focused on the use of live strains of crippled *Shigella* bacteria which will not replicate efficiently in the tissue of the host, or which are auxotrophic for aromatic compounds, or hybrids of the less invasive *Escherichia coli* and *Shigella* (Kärnell et al., *Vaccine,* 13:495-502, 1995). However, a satisfactory level of immune response in the vaccinated subject is still difficult to obtain while maintaining a sufficient margin of safety. This obstacle has also prevented the formulation of an effective *Salmonella* vaccine. Thus, the need for effective, mass-producible vaccines for shigellosis, salmonellosis, and against enteroinvasive *E. coli* still exists.

There also exists a need for effective and safe adjuvants to boost the immunogenicity of other vaccine preparations. Many proteins, carbohydrates, and even nucleic acids are not able to induce an immune response in animals unless they are coadministered with an adjuvant. An adjuvant is an agent that increases specific immune responses to a coadministered antigen. There are several types of adjuvants, including aluminum salts (alum), cytokines, surface active agents, and various bacterial products such as Freund's complete adjuvant or *V. cholerae* and *E. coli* enterotoxins.

Effective adjuvants that are safe for use in humans or animals are necessary for the development of more effective vaccines. Several virulent organisms are too dangerous to administer as live vaccines, even in the form of attenuated strains. However, heat-inactivated viruses or bacteria, or recombinantly produced constituents thereof, are not always immunogenic enough to elicit the strong immune response necessary for protective immunity. The use of adjuvants can enhance both the immediate immune response that an animal generates to the antigen component of the vaccine, and extend the duration of its effectiveness. Freund's adjuvant has been known to cause chronic pain and cancer in subjects, and is thus not suitable for use in a vaccine. Likewise, the bacterial toxin adjuvants are not currently approved for human use. Cytokine preparations and others are currently in evaluation for their safety. The only adjuvant currently approved for use in human preparations is alum, which exhibits only weak adjuvanticity.

Effectiveness of an adjuvant is often dependent upon its ability to stabilize epitope conformation, preserve the antigen from rapid clearance and degradation, and to target the antigen to surface receptors on antigen-presenting cells. Many adjuvants accomplish this function by acting as depositories which slowly release the antigen thereby continuously challenging the immune system over a period of time. Freund's complete and incomplete mineral oil emulsion adjuvants and alum (which traps antigens in an aluminum gel matrix) act in this fashion. Other adjuvants stimulate the proliferation and activation of lymphocytes in the immediate area of the antigen administration. Interleukin 12 and 15, and other cytokine adjuvants, function in this manner (U.S. Patents Nos. 5,723,127 and 5,747,024). Many very effective adjuvants stimulate a strong immuno-response to themselves, which encompasses an immuno-response to the coadministered antigen. These adjuvants include various bacterially derived toxins (U.S. Patent No. 5,182,109), and the mycobacterium component in Freund's complete adjuvant.

The mucosal immune system is distinct from the peripheral immune system. In the peripheral immune system, lymphoid cells and effector molecules are confined to individual lymph nodes and the spleen and intercommunication occurs by cell trafficking through the lymphatic and blood circulation. In contrast, the mucosal immune system is an integrated network of tissues, lymphoid and constitutive cells, and effector molecules which protect the host from infection of the mucous membrane surfaces. Because stimulation of the peripheral immune system does not result in significant mucosal immunity, protection against organisms which attack the mucosa requires stimulation of the mucosal immune system.

The importance of the mucosal immune system, however, extends far beyond protection against organisms such as *Shigella, Salmonella* and enteroinvasive strains of *E. coli* that directly attack the mucosa of the intestine. The use of vaccines to stimulate mucosal immune responses is especially attractive considering that most infectious agents first come in contact with the host at mucosal surfaces. Thus, induction of mucosal immune responses may not only protect the host from morbidity and mortality due to infection, but can possibly prevent infection altogether. In addition, unlike the peripheral immune system, where stimulation does not result in a corresponding mucosal response, induction of mucosal immune responses can also result in stimulation of protective immunity in the peripheral system.

Of all the established adjuvants, only cholera toxin (CT) and the *E. coli* heat labile toxin (LT) are purified proteins capable of inducing a potent systemic and mucosal immune response. Cholera toxin acts to stimulate a mucosal compartment response through induction of T helper 2 (Th2) cells (Marinaro et al., *J. Immunol.* 155:4621-29, 1995). T helper cells, in turn, act to stimulate B cells through the production of cytokines. Th2 cells are characterized by the production of the interleukins (IL) IL-4, IL-5, IL-6, IL-10 and IL-13. Th2 cells are considered bo be the major helper phenotype for support of IgG1, IgE and IgA secretion by B cells.

The ability to stimulate a mucosal immune response is a highly desirable property, as most adjuvants are used primarily for systemic immunizations and produce little to no secretory immunity. Such immunity is important for protecting against infective entities which first attack epithelial cells, such as *Shigella* or *Streptococcus pneumoniae.* Unfortunately CT and LT are toxic molecules and have required genetic modifications to render these adjuvants relatively safe and effective. CT subunit B (CTB) has been produced recombinantly, and exhibits significantly reduced toxicity in comparison to its parent. CTB is usually chemically or genetically coupled to the antigen of interest in order to invoke the desired immune response. Recently, Oaks et al., have used fractions of a water extract of *Shigella* as an adjuvant and in vaccine preparations (U.S. Patent Application Nos. 60/102,397, 60/102,398, 60/136,190). These fractions, termed Invaplex 24 and Invaplex 50, contained various Ipa proteins and lipopolysaccharide (LPS).

Purification of the IpaC protein has proven difficult. The physical and chemical characteristics of IpaC make it highly adherent to other proteins, and itself, under a variety of conditions. Thus, although several strategies have been tried, ranging from deleting contaminant protein genes in the native *Shigella* organism, to utilizing several commercial purification systems on the market, until this point high purity, high quantity purification of IpaC, and similar invasins, has proven elusive. De Geyter et al., *FEBS Lett*., 400:149-154, 1997, reported the purification of the IpaC protein to over 90% purity could be accomplished in the presence of a protein denaturant (4 M urea). In the absence of urea, the IpaC was found in several fractions. The need for a denaturant to maintain solubility of the IpaC makes this preparation unsuitable for pharmaceutical uses and may limit the biological function of the protein compared to its non-denatured, native state. Marquart et al., *Infect. Immun*., 64:4182-4187, 1996, and Picking et al., *Prot. Express. Purificat.,* 8:401-408, 1996, reported the purification to greater than 90% of a recombinant IpaC protein containing a poly histidine affinity purification moiety. Preparations of greater purity, however, have not been reported prior to the present invention. The high degree of purity achieved by the present invention is due to the unique combination of affinity purification in the presence of a denaturant, followed by rapid removal of the denaturant. Although the use of either denaturants or affinity purification of IpaC proteins had been known in the art, the combination of the two had not been used. More importantly, the prior art does not teach the rapid dilution of the denaturant necessary to maintain the solubility of the highly purified Invasin protein.

### SUMMARY OF THE INVENTION

The invention is defined in the accompanying claims.

Generally, the present invention comprises purified recombinant invasin proteins, methods for producing purified recombinant invasin proteins and use thereof. More specifically, there is described a substantially purified recombinant invasin protein, said protein comprising an amino acid sequence derived from one of the invasin proteins of a *Shigella* spp., *Salmonella spp.*, or enteroinvasive *E. coli* bacterium. Such invasins include the IpaC and SipC proteins. Also included are purified recombinant invasin proteins wherein said proteins have adjuvant activity.

The purification method described herein is superior to previously known methods in that it allows the production of fully soluble, biologically active invasin proteins which are substantially free of denaturants and are at least 95% pure. Such highly purified invasin proteins have multiple uses. Because of their high purity and ease of production, the invasin proteins described herein provide a means to meet the need for effective, mass-producible vaccines for shigellosis, salmonellosis and enterinvasive *E*. *coli.* In addition, the ability of the invasin proteins described herein to elicit an immune response makes them useful as adjuvants. Such adjuvant preparations are useful in the prevention of disease and in stimulating the immune system of immunocompromised individuals. As adjuvants, the high purified recombinant proteins described herein are superior to presently approved adjuvants due to their low toxicity and their ability to stimulate both peripheral and mucosal immune responses. Presently available adjuvant preparations either fail to elicit a strong mucosal immune response or present toxicity problems. In addition, the ability of the highly purified recombinant invasin proteins described herein to stimulate cell phagocytosis, makes them useful for the intracellular delivery of therapeutic and diagnostic agents.

Accordingly, a composition is described herein comprising a recombinant invasin protein of at least 95% purity. In one embodiment the purified invasin protein comprises an amino acid sequence derived from a portion of an invasin protein from a *Shigella* spp., *Salmonella* spp., or enteroinvasive *E. coli* of at least 15 amino acids in length and in which no more than 35% of the amino acid residues have been conservatively substituted.

Also described herein is a method of producing a substantially purified invasin protein comprising:
a) combining a polynucleotide encoding the invasin protein and a polynucleotide encoding an affinity purification moiety;
b) transforming the combination of a), in an appropriate expression vector, into a host cell;
c) growing the host cell under conditions conducive to soluble protein expression;
d) extracting the protein from a host cell lysate, culture medium, or reconstituted organism with a solution comprising a protein denaturant;
e) performing an affinity purification of the invasin protein appropriate for the affinity purification moiety encoded by the polynucleotide in a), wherein the method of said purification is performed in the presence of a protein denaturant;
f) removing said protein denaturant from the protein solution obtained in the purification process of e) until the concentration of the denaturant is at the minimum concentration necessary to maintain protein solubility; and
g) rapidly diluting the purified protein into a volume of denaturant-free solution.

The present invention is directed to an adjuvant composition comprising a purified recombinant invasin protein, wherein administration of the adjuvant composition in combination with an antigen to an animal elicits an immune response to the antigen. Such mucosal immune response can be characterized by the production of specific antibodies of the IgG, IgM, IgA and IgE classes, or by activated Tcell which produce cytokines, including IL-4, IL-5, IL-6, IL-10 and IL-13. In one embodiment, the purified recombinant invasin protein of the adjuvant composition is derived from a protein of a member of the *Shigella* or *Salmonella* genus, or from an enteroinvasive *E. coli*. Such adjuvant compositions may further comprise other adjuvants and immune system stimulants, including, but not limited to, cytokines and alum.

Another aspect described herein is a vaccine composition comprising a purified recombinant invasin protein having adjuvant activity, at least one antigen and a pharmaceutically acceptable carrier, diluent or excipient wherein administration of the vaccine composition elicits an immune response directed at the antigen(s) that involves T cells, B cells, synthesis of antigen-reactive antibodies or all three.

A further aspect described herein is a vaccine for eliciting an immune response against an organism expressing invasin protein antigens comprising a purified recombinant invasin protein having adjuvant activity derived from the invasin protein antigen expressing organism against which immunity is desired. The immune response elicited is directed specifically at the invasin and involves T cells, B cells or both.

Also described herein is a method for the delivery of pharmacologically active substances, therapeutic substances, cytotoxic substances, or diagnostic substances into cells comprising administering a pharmacologically active substance, cytotoxic substance, or diagnostic substance and a purified recombinant invasin protein.

Although primarily directed at stimulating immune responses in animals, the compositions and methods of the present invention can be used to stimulate immune responses by cells *in vitro*.

### BRIEF DESCRIPTION OF THE DRAWINGS

These and other features, aspects, and advantages of the present invention will become better understood with regard to the following description, appended claims, and accompanying drawings where:
- FIG. 1: shows a schematic of a linearized plasmid pET15b containing a DNA sequence encoding a recombinant invasin protein (IpaC or SipC).
- FIG. 2: shows IgA serum immunity results of an experiment in which groups of mice were intranasally immunized repeatedly with ovalbumin coadministered with cholera toxin (CT) or recombinant IpaC.
- FIG. 3: shows IgG serum immunity results of an experiment in which groups of mice were intranasally immunized repeatedly with ovalbumin coadministered with cholera toxin (CT) or recombinant IpaC.

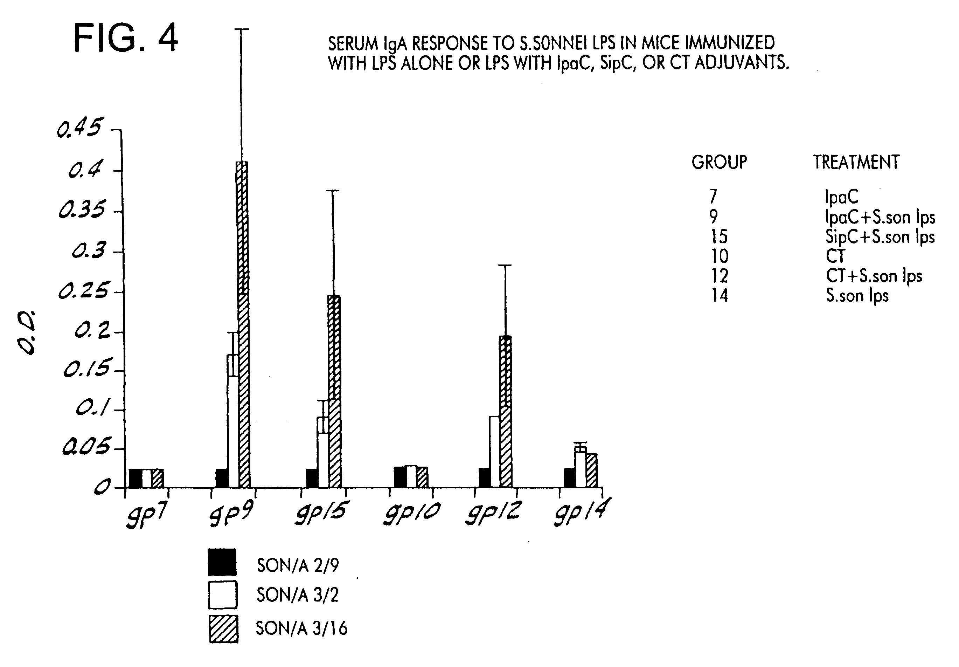

- FIG. 4: shows IgA serum immunity results of an experiment in which groups of mice were intranasally immunized repeatedly with *Shigella sonnei* lipopolysaccharide coadministered with cholera toxin (CT) or recombinant IpaC or SipC.
- FIG. 5: shows an SDS-PAGE gel of IpaC-HisTag and SipC-HisTag proteins produced as described in Example 1. Lane 1 contains molecular weight standards. Lane 2 contains the whole extract of induced cells. Lane 3 contains the flow through from the His-Bind® affinity purification column. Lanes 4-10 contain fractions of the eluate from the affinity purification column. As can be seen by the lack of contaminating proteins, these recombinant invasin proteins are at least 95% pure.
- FIG. 6: shows IgG subclasses elicited by intranasal immunization of mice with Shigella IpaC or cholera toxin (CT) mixed with ovalbumin (OVA). Immunization with either IpaC plus ovalbumin (IpaC/OVA), or cholera toxin plus ovalbumin (CT/OVA) are indicated as treatments. The optical density (O.D.) was measured in an ELISA assay for each of the four immunoglobulin G subclasses (IgG1, IgG2a, IgG2b, and IgG3). Serum for this analysis was collected 2 weeks after the last immunization. The mean OD ± SEM is plotted for each IgG subclass.
- SEQ. ID NO. 1: is the native amino acid sequence of the SipC protein *of Salmonella typhimurium.*
- SEQ. ID NO. 2: is the native amino acid sequence of the IpaC protein of *Shigella flexneri.*

### DEFINITIONS

As used herein, a "substantially purified protein" means that the protein is separated from the vast majority of host cell proteins normally associated with it or that the protein is synthesized in substantially purified form.

A "substantially isolated nucleic acid polymer" means that the mixture which comprises the nucleic acid polymer of interest is essentially free of a majority of other nucleic acid polymers normally associated with it. A "nucleic acid polymer" includes a polymer of nucleotides or nucleotide derivatives or analogs, including for example deoxyribonucleotides, ribonucleotides, etc. Genomic DNA, cDNA and mRNA are exemplary nucleic acid polymers.

The term "regulatory expression mechanism" is intended to include promotion and/or repression of transcription or mRNA or production/translation of a protein.

The term "gene" is intended to include both endogenous and heterologous genes, and specifically, both genomic DNA which encodes a target protein in a naturally occurring cell, and also cDNA encoding the target protein, for example, wherein the cDNA is a part of a nucleic acid construct such as a plasmid vector or virus which has been introduced into a cell or a cDNA produced by RT-PCR.

"Recombinant" means that the protein, whether comprising a native or mutant primary amino acid sequence, is obtained by expression of a gene carried by a recombinant DNA molecule in a cell other than the cell in which that gene and/or protein is naturally found. In other words, the gene is heterologous to the host in which it is expressed. It should be noted that any alteration of a gene, including the addition of a polynucleotide encoding an affinity purification moiety to the gene, makes that gene unnatural for the purposes of this definition, and thus that gene cannot be "naturally' found in any cell. For instance, IpaC modified to include an affinity purification moiety reinserted into and expressed in *Shigella* would still be considered to be recombinant. Likewise an invasin protein modified by the addition of a protein or protein fragment to produce a fused or hybrid protein would be considered recombinant.

A "fused" "fusion" or "hybrid" protein means a protein made up of at least two linked and different proeins produced from a fused gene.

A "fused gene" means a hybrid gene produced by linking two or more genes using methods of recombinant DNA technology.

A protein is "derived" from a protein in an organism when the wild-type protein on which the protein is based naturally occurs in that organism. For instance, IpaC incorporating a affinity purification moiety is derived from an invasin of *Shigella flexneri.*

An "affinity purification moiety," for the purposes of this invention, means moiety that has been added to a protein in order to allow the protein to be purified using some affinity purification scheme. This portion of the protein may or may not be cleaved from the protein after purification. An example of an affinity purification moiety is the poly-histidine nickel-chelating amino acid sequence described in U.S. Patent No. 5,594,115 and commercially available under the name His-Tag® (Novagen, Madison, WI).

A "denaturant," for the purposes of the invention, is a chemical substance which induces a conformational change in a protein, interfering with protein-protein intra-actions and causing it to lose its tertiary structure. Examples of denaturants are urea and detergents.

An "invasin" or "invasin protein", for the purposes of this invention, is a protein produced and secreted by an enteroinvasive bacteria which is necessary for that organism to induce phagocytosis in an epithelial cell.

The term "vector" is intended to include any physical or biochemical vehicle containing nucleic acid polymers of interest, by which those nucleic acid polymers are transferred into a host cell, thereby transforming that cell with the introduced nucleic acid polymers. Examples of vectors include DNA plasmids, viruses, particle gun pellets, and bacteria such as *Agrobacterium tumefaciens.* The term "primary vector" is intended to mean the first vector used in a transformation series, either as one step (e.g. a plasmid used to transform a yeast cell), or with a "secondary vector" (*e*.*g*. a plasmid used to transform *Agrobacterium tumefaciens,* which is later used to transform a plant cell).

The term "host cell" is intended to mean the target cell for vector transformation, in which the transferred nucleic acid polymer will be replicated and/or expressed.

The term "conservative substitution," in the context of amino acid sequences, means the substitution of one amino acid in the sequence with another with a side chain of similar size and charge. An example of a conservative substitution would be substituting glutamine for asparagine. Conservative substitutions in a protein sequence which would be expected to have minimal to no impact on protein structure or function can be readily devised by a person of ordinary skill in the biochemical arts.

The term "animals" is intended to include human beings.

A substance "elicits" or "stimulates" an immune response when it causes a change in immune status. Thus, a substance can be said to elicit or stimulate an immune response when it results in a de novo immune response or alters an already existing immune response.

### DETAILED DESCRIPTION OF THE INVENTION

The present specification describes for a purified recombinant invasin protein and novel methods for the preparation of same. Also described are purified recombinant invasin proteins with adjuvant activity and methods for their preparation and use. Thus, the purified recombinant invasin protein is preferably a susbtantially purified recombinant invasin protein. The invasin protein is preferably over 95 % pure, most preferably over 97% pure, purity being defined as the absence of contaminating proteins, nucleic acids, and other biologicals. The presence of contaminating proteins can readily be determined by one- or two-dimensional electrophoresis, such as SDS-PAGE analysis, or by other techniques well known to the person of ordinary skill in the art, for example, but not limited conventional chromatography and high performance liquid chromatography (HPLC).

The present invention also provides for a novel adjuvant that induces an immune response comprising a purified invasin, pharmaceutical compositions containing said adjuvant, and novel methods of the preparation thereof.

Unexpectedly, it has been found that administration of purified recombinant invasin proteins to mice mimics the immune response induced by the use of cholera toxin as an adjuvant. In particular, intranasal administration of recombinant invasin protein IpaC in mice resulted in a substantial increase in the production of a predominately IgG1 subclass. These data indicate an IL4/Th2 driven immune response which is characteristic of induction of a mucosal immune response. Induction of a mucosal immune response is critical to the development of a successful vaccine against organisms whose target is the mucosal layer such as members of the genera *Shigella* and *Salmonella* and enteroinvasive strains of *Escherichia coli.*

Adjuvants that stimulate a mucosal immune response to an antigen are superior to other adjuvants. Since most infectious agents first come in contact with the host at mucosal surfaces, induction of mucosal immune responses may not only protect the host from morbidity and mortality due to infection, but can possibly prevent infection altogether. Additionally, materials that stimulate a mucosal response are superior because of their potential to induce an immune response in both the mucosal and peripheral immune compartments. In contrast, presently available adjuvants elicit an immune response to an antigen only in the peripheral compartment.

None of the adjuvants currently approved for use have been shown to be capable of eliciting an IL4/Th2 driven response. Thus, the present invention fulfills a critical need by providing an alternative method for inducing a mucosal immune response in animals.

Accordingly, there is described herein a purified recombinant invasin protein, which may be used advantageously as an adjuvant. The invasin protein is preferably over 95 % pure, most preferably over 97% pure.

In a preferred embodiment of the present invention, the recombinant invasin protein includes the amino acid sequence of SEQ ID NO. 2(IpaC) or SEQ ID NO. 1 (SipC), or that of the IpaC invasin protein of enteroinvasive *E. coli.* Other embodiments of the recombinant invasin proteins include the amino acid sequence of the IpaC protein of *Shigella boydii, Shigella dysentariae, Shigella sonnei,* or another *Shigella* spp. Another embodiment includes the SipC protein of *Salmonella typhi* or another enteroinvasive *Salmonella* spp. Those of ordinary skill in the art are aware that modifications in the amino acid sequence of a peptide, polypeptide, or protein can result in equivalent, or possibly improved, second generation peptides, etc., that display equivalent or superior functional characteristics when compared to the original amino acid sequence. The present invention accordingly encompasses such modified amino acid sequences. Alterations can include amino acid insertions, deletions, substitutions, truncations, fusions, shuffling of subunit sequences, and the like, provided that the peptide sequences produced by such modifications have substantially the same functional properties as the naturally occurring counterpart sequences disclosed herein. Thus, for example, modified recombinant invasin proteins should possess substantially the same adjuvant activity as the naturally occurring counterpart sequence.

One factor that can be considered in making such changes is the hydropathic index of amino acids. The importance of the hydropathic amino acid index in conferring interactive biological function on a protein has been discussed by Kyte and Doolittle (*J. Mol. Biol.*, 157: 105-132, 1982). It is accepted that the relative hydropathic character of amino acids contributes to the secondary structure of the resultant protein. This, in turn, affects the interaction of the protein with molecules such as enzymes, substrates, receptors, DNA, antibodies, antigens, etc.

Based on its hydrophobicity and charge characteristics, each amino acid has been assigned a hydropathic index as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate/glutamine/aspartate/asparagine (-3.5); lysine (-3.9); and arginine (-4.5).

As is known in the art, certain amino acids in a peptide or protein can be substituted for other amino acids having a similar hydropathic index or score and produce a resultant peptide or protein having similar biological activity, i.e., which still retains biological functionality. In making such changes, it is preferable that amino acids having hydropathic indices within ±2 are substituted for one another. More preferred substitutions are those wherein the amino acids have hydropathic indices within ±1. Most preferred substitutions are those wherein the amino acids have hydropathic indices within ±0.5.

Like amino acids can also be substituted on the basis of hydrophilicity. U.S. Patent No. 4,554,101 discloses that the greatest local average hydrophilicity of a protein, as governed by the hydrophilicity of its adjacent amino acids, correlates with a biological property of the protein. The following hydrophilicity values have been assigned to amino acids: arginine/lysine (+3.0); aspartate/glutamate (+3.0 ±1); serine (+0.3); asparagine/glutamine (+0.2); glycine (0); threonine (-0.4); proline (-0.5 ±1); alanine/histidine (-0.5); cysteine (-1.0); methionine (-1.3); valine (-1.5); leucine/isoleucine (-1.8); tyrosine (-2.3); phenylalanine (-2.5); and tryptophan (-3.4). Thus, one amino acid in a peptide, polypeptide, or protein can be substituted by another amino acid having a similar hydrophilicity score and still produce a resultant protein having similar biological activity, i.e., still retaining correct biological function. In making such changes, amino acids having hydropathic indices within ±2 are preferably substituted for one another, those within ±1 are more preferred, and those within ±0.5 are most preferred.

As outlined above, amino acid substitutions in the proteins described herein can be based on the relative similarity of the amino acid side-chain substituents, for example, their hydrophobicity, hydrophilicity, charge, size, etc. Exemplary substitutions that take various of the foregoing characteristics into consideration in order to produce conservative amino acid changes resulting in silent changes within the present proteins can be selected from other members of the class to which the naturally occurring amino acid belongs. Amino acids can be divided into the following four groups: (1) acidic amino acids; (2) basic amino acids; (3) neutral polar amino acids; and (4) neutral non-polar amino acids. Representative amino acids within these various groups include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, cystine, tyrosine, asparagine, and glutamine; and (4) neutral non-polar amino acids such as alanine, leucine, isoleucine, valine, proline, phenylalanine, tryptophan, and methionine. It should be noted that changes which are not expected to be advantageous can also be useful if these result in the production of functional sequences.

To the extent that such conservative substitutions can be made while retaining 65% or more identity to SEQ. ID NO. 1 or 2, or one of the other invasins mentioned above, and also retaining adjuvant activity, such altered proteins are within the scope of the present invention. Thus, the invention may utilise proteins which have adjuvant activity and have at least about 65% sequence identity to SEQ ID NO. 1 or SEQ ID NO. 2, or one of the other invasins mentioned above, and more preferably at least about 90% sequence identity to one of the listed invasins, with the remaining amino acids being conservatively substituted. In other embodiments, the recombinant invasin protein may comprise an amino acid sequence of another invasin of *Shigella* spp., *Salmonella* spp., or enteroinvasive *E. coli.*

A sufficient degree of adjuvant activity may be obtained by using a protein which contains the adjuvanticity domain of either the SipC or IpaC. As has been demonstrated for the B subunit of cholera toxin (CT), particular portions of an protein may function as adjuvants when excised from the remainder of the protein. Therefore, the invention is also directed to a purified recombinant invasin protein which has adjuvant activity and which includes a portion of the amino acid sequence of SEQ ID NO. 1 or SEQ ID NO. 2, or one of the other invasins mentioned above, of at least about 15 amino acid residues in length, more preferably about at least about 20 amino acids in length, most preferably about at least 30 amino acid residues in length, where the included portion of the invasin confers the adjuvant activity on the protein. Examples 3 and 4 illustrate the process of selecting such a portion of the amino acid sequence for use in a recombinant invasin adjuvant protein

Methods for producing recombinant proteins in which portions of the native amino acid sequence have been deleted are known to those of ordinary skill in the art, and can be found in, for example, Davis et al., *Basic Methods in Molecular Biology,* Elsevier Scientific Publishing, 1986, Sambrook et al., *Molecular Cloning, A Laboratory Manual,* 2^{nd} Ed., Cold Spring Harbor Press, 1989 Watson et al., *Recombinant DNA,* 2^{nd} ed., Scientific American Books, 1992 and Ausubel et al., *Short Protocols in Molecular Biology,* 2^{nd} Ed., John Wiley & Sons 1992. In one such method to create end deletions, a plasmid containing the nucleotide sequence for the protein is linearized by treatment with a restriction enzyme. The ends of the linearized sequence are then digested by use of a exonuclease. Oligonucleotide linkers incorporating suitable restriction enzyme sites are then added to the digested sequence using DNA ligase. The restriction sites in the linkers are then used to insert the digested sequence into a plasmid. If a unilateral deletion is desired, then an exonuclease such as Exonuclease III, which preferential digests the 3' end of a linear DNA sequence is used.

In another method, restriction enzyme digestion can be used to create deletions on the ends or in the middle of a sequence. In this method, restriction enzymes are used to create two cuts, either within the sequence or one cut within the sequence and another cut flanking the sequence. The plasmid can then be treated with S1 nuclease to create blunt ends and the ends ligated with DNA ligase. The resulting plasmid contains the original sequence minus that part of the sequence flanked by the restriction sites.

Deletion mutations can also be created using the polymerase chain reaction (PCR). Methods for conducting PCR reactions are well known to those of ordinary skill in the art (U.S. Patent Nos. 4,683,195 and 4,683,202 and Innis et al., *PCR Protocols,* Academic Press, 1990). In one method to create end deletions, primers are designed so that one primer flanks the sequence of interest while the other primer binds within the sequence itself. The primers are designed to incorporate restriction enzyme sites to allow insertion of the amplified sequences into a vector. The selected sequence is then amplified by PCR and the amplification products purified and inserted into a suitable vector. If a deletion within the sequence is desired, two sets of primers are used. For each set of primers, one primer flanks the sequence of interest while the other primer binds within the sequence, but does not overlap the sequence flanked by the other primer pair. Again, restriction sites are incorporated into the primers. In this case, the primers that bind within the sequence contain the same restriction site so that the amplification products can be ligated. The sequences are then amplified using PCR. The amplification can take place in a single reaction using both sets of primers, or in two separate reactions. After purification, the amplification products are treated with the proper restriction enzyme and ligated together. The ligated amplification products are then inserted into a suitable vector. Using this method, that portion of the sequence not flanked by the primers is deleted.

As mentioned above, one of ordinary skill in the biochemical arts may devise several conservative substitutions in an amino acid sequence, allowing creation of a peptide with a different primary amino acid structure which still retains the functionality of the original peptide. Thus, the invention is also directed towards proteins which have adjuvant activity and include a portion of at least about 15 amino acid residues in length, more preferably at least about 20 amino acids in length, even more preferably at least about 30 amino acid residues in length, with at least about 65% sequence identity to a similarly sized portion of SEQ ID NO. 1 and SEQ ID NO. 2, or one of the other invasins mentioned above, and more preferably at least about 95% sequence identity to a similarly sized portion of one of the listed invasins with the remaining amino acids being conservatively substituted.

Another aspect described herein is a method of producing the substantially purified recombinant invasin protein of the invention by affinity purification. Affinity purification is based on the specific affinity between the molecule to be isolated and a molecule that it can bind (a ligand). The binding of the molecule to the ligand can involve biochemical or immuno-chemical interactions. The ligand is linked to an insoluble support in a manner that does not destroy its binding activity and specificity. When necessary, a spacer may be inserted between the ligand and the support to prevent steric hindrance. When the molecule of interest is brought in contact with the ligand, for example in a affinity chromatography column, the molecule binds to the ligand. Elution is achieved by changing the conditions so that binding of the molecule and the ligand no longer occurs. The basic method comprises the following steps:
a) inserting a polynucleotide encoding an invasin protein into an expression vector;
b) transforming the combination of a) into a host cell;
c) growing the host cell under conditions conductive to soluble protein expression;
d) extracting the protein from a host cell lysate, culture medium, or reconstituted organism with a solution comprising a protein denaturant;
e) performing an affinity purification of the invasin protein wherein the method of said purification is performed in the presence of a protein denaturant;
f) removing said protein denaturant from the protein solution obtained in the purification process of e) until the concentration of the denaturant is at the minimum concentration necessary to maintain protein solubility; and
g) rapidly diluting the purified protein into a volume of denaturant-free solution.

It will be readily apparent to those of ordinary skill in the art that the above procedure may be modified to fit a particular use. Such modifications may be made following routine experimentation to obtain the desired result and are within the scope of the present invention. For example, and without limitation, one may wish to further reduce the denaturant concentration in the product resulting from the above procedure by dialysis or ultrafiltration against a buffer suitable for injection into an animal.

Any affinity purification system that functions under denaturing conditions can be used to isolate the recombinant invasin protein of the current invention. Affinity purification systems for recombinant proteins typically involve production of a fusion protein comprising the protein of interest and a ligand capable of binding with high specificity to an affinity matrix. A review of various methods for the affinity purification of recombinant fusion proteins can be found in U.S. Patent No. 5,935,824. Accordingly another aspect described herein is a method for producing a substantially purified recombinant invasin protein of the invention using an affinity purification moeity comprising:
a) combining a polynucleotide encoding an invasin protein and a polynucleotide encoding an affinity purification moiety;
b) transforming the combination of a), in an appropriate expression vector, into a host cell;
c) growing the host cell under conditions conductive to soluble protein expression;
d) extracting the protein from a host cell lysate, culture medium, or reconstituted organism with a solution comprising a protein denaturant;
e) performing an affinity purification of the invasin protein appropriate for the affinity purification moiety encoded by the polynucleotide in a), wherein the method of said purification is performed in the presence of a protein denaturant;
f) removing said protein denaturant from the protein solution obtained in the purification process of e) until the concentration of the denaturant is at the minimum concentration necessary to maintain protein solubility; and
g) rapidly diluting the purified protein into a volume of denaturant-free solution.

Although the presence of affinity binding ligands in recombinant fusion proteins may be extremely useful for purification, the presence of the ligand may alter the activity of the recombinant protein of interest or have other undesirable aspects. In such cases it is desirable to be able to separate the protein of interest from the rest of the fusion protein. Typically in such cases a tripartite fusion protein is formed in which a site for proteolytic or chemical cleavage is inserted between the affinity purification ligand and the protein of interest. A review of such systems can be found in U.S. Patent No. 5,935,824, hereby fully incorporated by reference, and references cited therein.

Of particular use in the present invention are affinity purification systems based on fusion proteins which contain metal chelating amino acid sequences. In a preferred embodiment, the affinity purification method used is the procedure disclosed in U.S. Patent No. 5,594,115 (herein incorporated by reference in its entirety) utilizing a poly-histidine nickel-chelating amino acid sequence and commercially available under the name His-Tag® (Novagen, Madison, WI). The six histidines of the His-Tag® peptide reversibly bind to chelated Ni²⁺ on a solid support, usually a chromatographic column matrix. The solid support may then be washed under stringent conditions in order to remove all contaminating proteins, allowing the protein of interest to be eluted with high selectivity. An entire system for the purification of proteins by this method, including vectors suitable for expression in *E*. *coli*, is available from Novagen, Madison, WI, under the name pET Expression Systems®. Also available from Novagen are affinity purification systems utilizing a peptide ligand of a ribonuclease (S-Tag ™), peptides recognized by specific bound antibodies (T7-Tag®, and HSV-Tag ®), and a peptide that binds to cellulose (CBD-Tag™). In addition, Promega, Madison, WI, offers , the PinPoint™ purification system, which utilizes a peptide which binds to biotin *in vivo,* which then binds as a complex to an streptavidin-coated matrix. All of these commercially available systems can be used in affinity purification of the recombinant invasin proteins of the present invention. In all of these systems, the peptide of the affinity purification moiety may be cleaved from the refolded, purified protein produced by the method of the invention by following the manufacturer's instructions.

As indicated, for expression in *E. coli,* many of the above affinity purification moieties come from the manufacturer pre-inserted into a suitable expression vector. The criteria for the selection of an appropriate vector include high copy number and retention by the host cell, the presence of sufficient markers for easy transformant selection, and the integration of an inducible promoter or constitutive promoter, as indicated. Usually, an inducible promoter will be desired in cell culture settings, and a constitutive promoter will be more desirable in whole organism production, as noted below.

Suitable expression vectors include chromosomal, non-chromosomal and synthetic DNA sequences, for example, SV 40 derivatives; bacterial plasmids; phage DNA; baculovirus; yeast plasmids; vectors derived from combinations of plasmids and phage DNA; and viral DNA such as vaccinia, adenovirus, fowl pox virus, and pseudorabies. In addition, any other vector that is replicable and viable in the host may be used.

The nucleotide sequence of interest may be inserted into the vector by a variety of methods. In the most common method the sequence is inserted into an appropriate restriction endonuclease site(s) using procedures commonly known to those of ordinary skill in the art and detailed in, for example, Sambrook et al., *Molecular Cloning, A Laboratory Manual,* 2^{nd} Ed., Cold Spring Harbor Press, (1989) and Ausubel et al., *Short Protocols in Molecular Biology,* 2^{nd} Ed., John Wiley & Sons (1992).

In an expression vector, the sequence of interest is operably linked to a suitable expression control sequence or promoter recognized by the host cell to direct mRNA synthesis. Promoters are untranslated sequences located generally 100 to 1000 base pairs (bp) upstream from the start codon of a structural gene that regulate the transcription and translation of nucleic acid sequences under their control. Promoters are generally classified as either inducible or constitutive. Inducible promoters are promoters that initiate increased levels of transcription from DNA under their control in response to some change in the environment, e.g. the presence or absence of a nutrient or a change in temperature. Constitutive promoters, in contrast, maintain a relatively constant level of transcription.

A nucleic acid sequence is operably linked when it is placed into a functional relationship with another nucleic acid sequence. For example, DNA for a presequence or secretory leader is operatively linked to DNA for a polypeptide if it is expressed as a preprotein which participates in the secretion of the polypeptide; a promoter is operably linked to a coding sequence if it affects the transcription of the sequence; or a ribosome binding site is operably linked to a coding sequence if it is positioned so as to facilitate translation. Generally, operably linked sequences are contiguous and, in the case of a secretory leader, contiguous and in reading phase. Linking is achieved by ligation at restriction enzyme sites. If suitable restriction sites are not available, then synthetic oligonucleotide adapters or linkers can be used as is known to those skilled in the art. Sambrook et al., *Molecular Cloning, A Laboratory Manual,* 2^{nd} Ed., Cold Spring Harbor Press, (1989) and Ausubel et al., *Short Protocols in Molecular Biology,* 2^{nd} Ed., John Wiley & Sons (1992).

Common promoters used in expression vectors include, but are not limited to, LTR or SV40 promoter, the E. coli lac or trp promoters, the T7 promoter, and the phage lambda PL promoter. Other promoters known to control the expression of genes in prokaryotic or eukaryotic cells can be used and are known to those or ordinary skill in the art. Expression vectors may also contain a ribosome binding site for translation initiation, and a transcription terminator. The vector may also contain sequences useful for the amplification of gene expression.

Expression vectors can and usually do contain a selection gene or selection marker. Typically, this gene encodes a protein necessary for the survival or growth of the host cell transformed with the vector. Examples of suitable markers include dihydrofolate reductase (DHFR) or neomycin resistance for eukaryotic cells and tetracycline or ampicillin resistance for E. coli.

As shown in the following examples, a preferred vector for use in the present invention is pET15b from Novagen (Madison, WI). However, if another host cell is used, the process of ligating polynucleotides encoding the invasin protein and the affinity purification moiety into an appropriate vector is routine to one of ordinary skill in the art of molecular biology without undue experimentation. Several vectors appropriate for expression in a wide variety of non-bacterial host cells (or bacterial cells other than *E. coli*) are available commercially and / or well known in the art. For instance, the pALTER®-MAX (which utilizes a human cytomegalovirus promoter) and pGL3 (which utilizes the SV40 promoter) from Promega are useful for expression of proteins in mammalian cells. The BacVector™ system from Novagen is particularly useful for expression in insect cells. *Agrobacterium* compatible vectors with a tobacco or cauliflower mosaic virus promoter is suitable for expression in a plant cell. One may select the particular vector and host cell for use in the present invention by considering a number of factors, including the availability of materials, preferred production methods (for instance, fermentation vats, versus growing transgenic crops, versus bleeding transgenic livestock), and the familiarity of the person of ordinary skill with a particular expression system.

The present invention relates to transformed host cells containing the constructs comprising the polynucleotide sequence encoding the recombinant invasin proteins of the present invention. The host cell can be a higher eukaryotic cell, such as a mammalian cell, or a lower eukaryotic cell such as a yeast cell, or the host can be a prokaryotic cell such as a bacterial cell. Introduction of the construct into the host cell can be accomplished by a variety of methods including calcium phosphate transfection, DEAE-dextran mediated transfection, polybrene, protoplast fusion, liposomes, direct microinjection into the nuclei, scrape loading, and electroporation.

A preferred host cell for use in the present invention is *E. coli* strain BL21 (DE3). However, as illustrated in the example, it may be necessary to transform the vector into another temporary host cell for the purposes of verifying the ligation product, increasing the amount of vector for later transfection of the host cell, or even as part of the host cell transfection process (such as when using an *Agrobacterium* mediated protocol to transform a plant host cell.) Two examples of how proteins like the recombinant invasin proteins of the present invention may be expressed in potato plants are Arakawa, et al. (*Transgenic Res.,* 6:403-413, 1997) and U.S. Patent No. 5,436,393, both incorporated herein by reference. Thus, the use of intermediate cell hosts for the amplification or transformation of DNA is within the present invention. Although a bacterial host is preferred for production of the recombinant invasin protein according to the method of the invention, primarily because eukaryotic expression of the protein may lead to undesirable glycosylation, production in eukaryotic cells, including mammalian, yeast, or plant cells, is within the scope of the present invention. Specifically, portions of SEQ ID NO. 1 and SEQ ID NO. 2 containing the antigenic epitopes discussed above may be recombined with other amino acid sequences and advantageously expressed in a eukaryotic cell in order to produce a recombinant protein adjuvant.

After transfection of the host cell with the vector containing polynucleotide sequences , the host cell is, according to the present invention, grown under conditions conducive to solubilizing the recombinant invasin protein. In preliminary experiments, the applicants encountered many problems when trying to express recombinant Ipa proteins. Expression of the recombinant form of IpaC derived from *S. flexneri* 2a was carried out using two different pET series vectors. pET15b was used to give a fusion product that possessed a six histidine ''tag'' as part of a short leader sequence at the N- terminus of the protein. Induction of pET15b-*ipaC* in *E*. *coli* BL21(DE3) at 37° C resulted in significant over expression of a 45-kDa protein, which was readily visible on Coomassie-stained SDS-polyacrylamide gels. Unfortunately, the majority of the induced protein (greater than 90%) was present as insoluble inclusions that remained with the cellular pellet following centrifugation.

In an attempt to increase the proportion of soluble IpaC expressed from pET15b, a variety of bacterial strains were transformed with pET15b-*ipaC*. These strains included *E. coli* BLR(DE3)pLysS, NovaBlue(DE3), and HMS174(DE3) (Novagen, Inc., Madison, WI) and were chosen based on the phenotypes of their recombination and methylation pathways and their ability to increase the solubility for some over expressed foreign proteins. Unfortunately, IpaC consistently partitioned to the insoluble cellular fraction for each strain tested.

As an alternative to expressing *ipaC* in different bacterial strains, the conditions used for growth of *E. coli* BL21(DE3) transformed with pET15b*-ipaC* were modified to slow the rate of bacterial growth and thereby reduce induction and synthesis of IpaC so that segregation of IpaC into inclusion bodies would be diminished. Control of the rate of growth and induction of gene expression was attempted using several different culture conditions such as reducing the amount of aeration in the culture and decreasing the growth temperature. Individually, these procedures resulted in only a minor increase in the proportion of soluble IpaC (data not shown); however, when the incubation temperature was reduced to 30° C and the shaking speed reduced from 250 to 150 rpm, the proportion of soluble IpaC was increased substantially. Thus, the preferred conditions for induction and production of the recombinant invasin proteins are growth at 30° C and slow shaking at 150 rpm, when the protein is produced in an *E. coli* strain. Similar hypoxic, hypothermic conditions may be used, upon optimization through routine experimentation, when utilizing the method of production with mammalian, yeast, or plant cell cultures, although the exact parameters may differ. When utilizing plant or animal cells which have been reconstituted into whole organisms, an alternative strategy may be more useful to slow the rate of protein synthesis. In these cases, down-regulatory genetic expression mechanisms, such as polynucleotides encoding antisense RNA complementary to that encoding the recombinant invasin protein which is regulated by a less active promoter, or the use of a vector comprising a weak or constitutive promoter, may be appropriate.

After allowing the production of the recombinant invasin protein in the host cells, the protein is purified according to a protocol appropriate for the affinity purification moiety employed in the method of the present invention, with the modification that all reagent solutions contain a protein denaturant. As the invasin adjuvant should be soluble in the cytosol of the host cell, or in the culture media if secreted, the supernatant should be used in the purification process once the cells or cell lysis debris have been pelleted by centrifugation. At this point, the denaturant should be added to the protein solution to an appropriate concentration. Preferred denaturants for use in the present invention include guanidine hydrochloride and urea. Although surfactants such as Tween and Triton may be used in the present invention, they are not preferred because of their tendency to form micelles, which are difficult to remove completely. The most preferable denaturant for use in the present invention is urea because of its efficacy as a denaturant and relatively low toxicity. The appropriate concentration for the denaturant in the protein solution is that concentration which will inhibit protein-protein interactions. For urea, this concentration is preferably between about 1 M and about 10 M, more preferably between about 5 M and about 7 M, and most preferably about 6 M. All solutions used in the purification process most preferably contain a denaturant at an appropriate concentration.

If the preferred His-Tag® affinity purification moiety is used, the protein may be purified on a His-Bind™ resin column under denaturing conditions according to the manufacturer's instructions, as outlined in the example. Denaturing protocols are also available for most of the affinity purification moieties listed above. If no denaturing protocol is provided for the particular affinity purification moiety chosen for use in the present invention, one may be easily formulated without undue experimentation by the practitioner of ordinary skill in the art by adding denaturant, to an appropriate concentration, to the solutions called for in the protocol.

After the protein has been purified, or at a later step, the affinity purification moiety may be selectively cleaved from the recombinant invasin protein, according to the manufacturer's directions for the particular affinity purification moiety. The Applicants have found that when the His-Tag® moiety is used, it may be left on the invasin protein adjuvant without affecting its adjuvant activity. However, the practitioner of ordinary skill in the art may decide to cleave the residue after routine experimentation to determine the protein's optimal adjuvanticity.

After the protein has been purified, but is still in a solution containing denaturant at an appropriate concentration, the protein must be refolded to regain its adjuvant characteristics. Conventional wisdom in the art of protein chemistry indicates that a stepwise or continuous removal of the denaturant is necessary to refold a protein. The Applicants surprisingly discovered that this procedure did not allow the refolding of the recombinant invasin proteins of the present invention without the formation of insoluble protein aggregates. After a certain minimum level of denaturant was reached, about 2 M for urea, the protein became insoluble. Thus, the Applicants had to devise a different approach. Surprisingly, the applicants found that a sudden dilution of the purified protein solution into a denaturant-free solution allowed the protein to refold without forming insoluble aggregates. Without limiting the invention to any particular theory or mechanism, applicants believe that the rapid removal of denaturant allows beneficial protein *intra-actians,* necessary for correct protein folding, to occur while the rapid dilution of the protein solution minimizes the probability of detrimental protein-protein *interactions,* which form aggregates. Therefore, the recombinant invasin protein, after affinity purification, is dialyzed into a buffer containing the minimum concentration of denaturant necessary to maintain solubility. If urea is used as the denaturant, this concentration is preferably from about 1 M to about 3 M, most preferably about 2 M. This buffer exchange may occur as a single step, a stepwise gradient, or a continuous gradient. The applicants have found that a buffer exchange as a single step is advantageous, as it saves considerable time. It should be noted that the invasin protein solution may be stored at this stage for later dilution and use.

The invasin protein solution, containing the minimum concentration of denaturant, is then rapidly diluted into a buffer containing no denaturant. This process is preferably completed in less than one minute, more preferably in less than 30 seconds, and most preferably in less than 10 seconds. A preferred buffer for use in the present invention is phosphate-buffered saline (PBS), although any other physiologically acceptable buffer would also be preferred. The denatured protein solution should be diluted into several times its volume of denaturant free buffer. The dilution ratio is preferably about 1 part denatured recombinant invasin protein solution to about 5 or more parts denaturant free buffer. The resultant solution contains biologically active, fully soluble recombinant invasin protein. The final concentration of the protein in solution is preferably about 1 mg per ml or less. It should be noted that the protein should not be further concentrated after dilution, as insoluble protein aggregates are likely to form.

The invention is also directed to adjuvant compositions comprising the recombinant invasin proteins of the present invention in a physiologically acceptable solution. An example of a preferred adjuvant composition of the present invention is the recombinant IpaC protein purified with a His-Tag® moiety, diluted into PBS, and further dialyzed against several volumes of PBS to remove the remaining denaturant. Preferably, the adjuvant composition comprises a recombinant invasin protein of at least 95% purity and more preferably of at least 97% purity. The adjuvant may be used alone as a vaccine in order to convey immunity against the organism of the wild type protein from which the protein is derived, or against a closely related organism. The adjuvant compositions of the present invention may also be advantageously used, alone or in combination with an antigen, to stimulate the immune response of individuals who are immunologically compromised because of age or immuno-suppression, or for other immuno-therapeutic uses for immuno-stimulatory compounds which have been described in the art, such as immunotolerization (see Czerkinsky et al., *Ann. N*.*Y*. *Acad. Sci*., 778:185-193, 1996). The immune response stimulated can involve T cells, B cells or both. When used for this purpose in combination with an antigen, the ratio of antigen to recombinant invasin protein is preferably about one part antigen to about 0.0001 to about 10,000 parts recombinant invasin protein, more preferably about one part antigen to about 0.001 to about 1,000 parts recombinant invasin protein and most preferably about one part antigen to about 0.01 to about 100 parts recombinant invasin protein.

The purified recombinant invasin proteins of the present invention may be mixed with antigens of biological or chemical origins to form a vaccine, and then administered to an animal to elicit an immune response to the antigen, as shown in the following examples. The immune response to the antigen can involve T cells, B cells or both. The antigen may be an infective agent, or a subunit thereof, or may be a biologically active chemical or toxoid. An infective agent can be a bacterium, virus, retrovirus, protozoan, parasite or fungus. Such a vaccine formulation, comprising recombinant invasin protein and an antigen of interest, is considered another aspect of the current invention. When used in vaccines, the recombinant invasin protein is preferably at least 95% pure and more preferably at least 97% pure. The recombinant invasin protein is also preferably combined with the antigen in a ratio of about one part antigen to about 0.0001 to about 10,000 parts purified recombinant invasin protein. More preferably, the recombinant invasin protein is preferably combined with the antigen in a ratio of about one part antigen to about 0.001 to about 1,000 parts invasin protein. Most preferably, the recombinant invasin protein is preferably combined with the antigen in a ratio of about one part antigen to about 0.01 to about 100 parts invasin adjuvant protein. Examples of preferred antigen to adjuvant ratios are the ovalbumin vaccine compositions of examples 2 and 4.

The adjuvants of the present invention exhibit several advantages over those currently available. Primarily, there exists a need for safe adjuvants which can stimulate mucosal immune activity directed toward a specific antigen. Although cholera toxin and heat-labile enteroinvasive *E. coli* are capable of stimulating a mucosal immune response, such adjuvants are toxic and cause noticeable distress in animals to which they are administered. In order to render them safer to use, such toxins must be genetically modified, a process that does not always preserve their full adjuvanticity. In fact, often an investigator will chemically conjugate or genetically fuse an antigen onto the genetically modified CT-B toxin in order to observe an adequate adjuvant effect. It should be noted that unlike these protein-adjuvants, the antigen of interest does not need to be chemically conjugated or genetically fused onto the recombinant invasin protein in order to obtain a potent adjuvant effect. Although cytokines, like Interleukin-12 and -15, are safer to use as adjuvants, they exhibit poor mucosal adjuvanticity. A sufficient secretory immune response is necessary in order to effectively immunize the subject animal against many diseases which first attack the mucus membranes. Although the adjuvants of the present invention are superior to those of the prior art, combinations of other adjuvants and those of the present invention are contemplated. Adjuvant compositions of the present invention further comprising cytokines or alum may exhibit a synergistic adjuvanticity.

In another aspect, the adjuvant or vaccine compositions of the present invention when administered to an animal elicit a specific immune response to an antigen. As discussed previously, an immune response is characterized by the stimulation of B cells through the production of cytokines by Th2 cells. Accordingly, administration of the adjuvant or vaccine compositions comprising the purified recombinant proteins of the present invention results in the production of cytokines by Th2 cells, more particularly interleukins (IL), and more particularly still the production of IL-4, IL-5, IL-6, IL-10 or IL-13. In another embodiment, the administration of adjuvant or vaccine compositions comprising the purified recombinant invasin protein of the present invention stimulates the production of IgG, IgE, IgM or IgA.

Administration of the adjuvants or vaccine compositions of the present invention can be accomplished by a variety of methods, including, without limitation, oral, enteral, mucosal, percutaneous, or parenteral. Examples of methods of administration include, oral, intranasal, intratracheal, intravenous, intramuscular, subcutaneous, intraperitoneal, intra-arterial, intrasternal, intralesional, topical, transdermal, inhalation and iontophoresis. Preferred methods of administration include intranasal, mucosal, oral, inhalation, rectal, vaginal, intratracheal and intestinal. As the adjuvants of the present invention advantageously stimulate a mucosal immune response, administration of the adjuvant or vaccines of the present invention is more preferentially by the intranasal route. Such administration may be made as a single dose or as a series of doses. For example, when using the recombinant invasin protein of the present invention to stimulate an immune response to ovalbumin, as in the examples below, several intranasal exposures over a series of weeks is desirable.

In yet another aspect the purified recombinant invasin protein can be used to deliver pharmacologically active substances, therapeutic substances, cytotoxic substances, diagnostic substances, etc., herein after commonly referred to as pharmacologically active substances, into cells. When used in this manner it is preferable that the invasin proteins be at least 95% pure and more preferably at least 97% pure. This aspect of the invention is based on the ability of the invasin proteins to cause pathogen induced phagocytosis. When used in this aspect of the invention, the purified recombinant invasin protein may be, but need not be, linked to the pharmacologically active substance. If desired, pharmacologically active substances can be linked to recombinant invasin proteins either by the production of fusion proteins or by coupling the pharmacologically active substance to the recombinant invasin protein either directly or through the use of a linker. Pharmacologically active substances can be coupled to either the amino- or carboxy-terminus of the purified recombinant invasin proteins. For example, drug conjugates wherein the carboxy terminus of a recombinant invasin protein is linked to a pharmacologically active substance can be prepared by the use of an active ester of the desired pharmacologically active substance in the presence of a dehydrating agent. Alternatively, a functional linker can be placed between the recombinant invasin protein and the pharmacologically active substance. A functional linker is a linker which can be cleaved, usually within a cell, to release the pharmacologically active substance from the recombinant invasin protein. Chemicals, reagents and techniques useful in drug cross-linking and peptide conjugation are disclosed in general texts well know to those skilled in the art, for example; Dawson, et al., (eds.), *Data for Biochemical Research,* 3rd Ed., Oxford University Press, Oxford, UK, 1986; King (ed.), *Medicinal Chemistry*: *Principles and Practice,* Royal Society of Chemistry, Cambridge, UK, 1994; Shan and Wong (eds.), *Chemistry of Protein Conjugation and Cross-Linking,* CRC Press, Boca Raton, 1991.

Alternatively, the pharmacologically active substance can be part of a fused protein comprising a recombinant invasin protein. A fused protein can be produced by methods well known to those skilled in the art of molecular biology. Briefly, a polynucleotide sequence encoding a pharmacologically active substance is linked to a polynucleotide encoding an invasin protein using standard molecular biology methods to create a fused gene. Davis et al., *Basic Methods in Molecular Biology,* Elsevier Scientific Publishing, 1986, Sambrook et al., *Molecular Cloning, A Laboratory Manual,* 2^{nd} Ed., Cold Spring Harbor Press, 1989 Watson et al., *Recombinant DNA,* 2nd ed., Scientific American Books, 1992 and Ausubel et al., *Short Protocols in Molecular Biology,* 2^{nd} Ed., John Wiley & Sons 1992. The fused gene is then inserted into a suitable expression vector which is in turn transfected into a host cell by methods described previously. The host cell then expresses the fusion protein either constitutively or in response to an inducer and the fusion protein is isolated as previously described.

The following examples illustrate the preparation of recombinant invasin antigen proteins of the invention, recombinant IpaC/HisTag®, and recombinant SipC\His-Tag ®, as well as the use of these proteins as adjuvants to stimulate immunity to an antigen. Also, the preparation of recombinant invasin proteins comprising portions of the IpaC protein are illustrated. These are merely exemplary of the principles and advantages of the invention, and are not intended to limit its scope in any way.

### EXAMPLES

### Example 1

### Production Purified of IpaC-HisTag and SipC-HisTag Recombinant Invasin Proteins

*S*. *flexneri* 2a was grown at 37° C with vigorous shaking in trypticase soy broth (TSB). The stock was maintained frozen at -70° C in 25% glycerol and 75% TSB. Prior to use, the bacteria were streaked onto trypticase soy agar (TSA) containing 0.025% Congo red so that colonies binding the dye could be selected. Bacteria that have lost the invasion plasmid are not able to bind this dye and thus appear white in the presence of Congo red. *Salmonella typhimurium* was grown under similar conditions.

Following transformation with the plasmids used here, *E. coli* transformants were selected on LB agar plates containing 50 µg/ml ampicillin. These strains were grown at 30° C with moderate shaking (approximately 150 rpm) in LB broth containing 50 µg/ml ampicillin to increase protein yield. All plasmid-bearing strains were stored at -70° C in LB containing 10% glycerol.

### Plasmid construction.

Isolation of plasmid DNA and all other molecular biology procedures were carried out according to standard published procedures. To confirm correct insertion of the desired fragments, plasmids were subjected to double-stranded DNA sequencing (Sequenase 2.0) according to the manufacturer's specifications. PCR primers to the 5' and 3' ends of the *IpaC* or *SipC* DNA sequences were produced based on their published sequences. Each 5' primer contained the sequence GAGA (SEQ ID NO: 3), an NdeI restriction site and 18 bases of the 5' end of each gene, respectively. Each 3' primer contained GAGA (SEQ ID NO: 3), a BamHI restriction site and 18 bases of the 3' end of each gene, respectively. Each sequence was amplified by PCR in a standard 100 µl reaction containing 2.5 mM MgCl₂, 0.25 mM of each dNTP, 100 pmol of the 5' and 3' primers, 10 µl boiled *S. flexneri* or S. *typhimurium,* and 5 U Taq DNA polymerase. Reactions were allowed to proceed in a Perkin-Elmer 480 thermal cycler programmed for 29 cycles (94° C, 45 sec; 63° C, 30 sec; and 72° C, 60 sec) with one additional cycle for 10 min at 72° C. Upon establishing that each PCR product was of the correct size by agarose gel electrophoresis, 7 µl of the reaction mixture was used directly for ligation of the fragment into the pCRII plasmid (Invitrogen, Inc., San Diego, CA) according to manufacturers specifications. The plasmids were then transformed into *E. coli* INVaF' and the transformants containing inserts identified by blue-white screening. The presence of the specific *IpaC* (SEQ ID NO. 2) or *SipC* (SEQ ID NO. 1) gene fragments was then confirmed by PCR using the conditions described above (except that 25 µl reactions were used with a T7 promoter forward primer and M13 reverse primer).

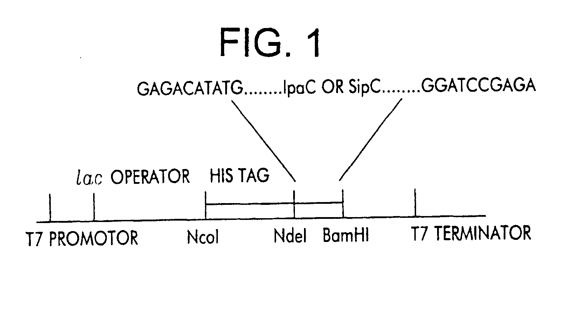

Plasmid DNA was purified and the fragments excised from the pCRII plasmid using NdeI and BamHI. These fragments were ligated into NdeI/BamHI-digested pET15b and the ligation products transformed into *E. coli* XL1B (Figure 1). Once again, transformants were screened for *IpaC* and *SipC*-sized inserts using PCR except that the forward and reverse primers were the T7 promoter and terminator sequences, respectively. Plasmid DNA was purified and used to sequence the cloning junctions by double-stranded DNA sequencing and to transform *E. coli* BL21(DE3). These plasmid-containing bacteria were then used for induction and subsequent purification of HisTag-Ipa or HisTag-Sip fusion protein products.

### Induction and purification of fusion proteins in the pET15b system.

Twenty ml of LB media containing 50 µg/ml ampicillin was inoculated with *E. coli* BL21(DE3) containing pET15b with one *IpaC* or *SipC* insert. The culture was grown overnight at 30°C with slow shaking (150 rpm). Increased protein yield was observed using the latter conditions. Four one liter flasks containing 400 ml LB supplemented with 50 µg/ml ampicillin were then inoculated with 5 ml of the overnight culture. Cultures were grown at either 37°C with vigorous shaking or 30°C with slow shaking until an absorbance of 0.3 to 0.45 at 550 nm was reached. Target protein synthesis was then induced by adding IPTG (isopropylthiogalactoside) to a final concentration of 1 mM. After a 3 h induction, the bacteria were quick chilled in an ice-water bath and collected by centrifugation at 8000 g for 10 min.

### Purification of IpaC and SipC with Urea

Recombinant IpaC and SipC could not be purified by following the manufacturer's normal protocol, as problems with maintaining IpaC or SipC in a soluble form at high concentrations required that the inventors modify the purification scheme for these proteins. Briefly, after induction of *IpaC* or *SipC* expression in *E. coli* BL21(DE3), the cells were harvested by centrifugation and the pellets resuspended in HisTag binding buffer containing 6 M urea. The cells were then frozen, quickly thawed, sonicated, and the solution clarified by centrifuging at 39,000 g for 20 min. The supernatant fraction could then be used for purification of IpaC and SipC by HisTag affinity chromatography as follows:

Affinity column chromatography using HisBind resin was performed at 4°C according to manufacturer's specifications (Novagen, Madison, WI), except that all buffers were augmented with 6 M urea. Briefly, 5 ml of HisBind resin in a 10 ml glass column was washed with 15 ml of water, 25 ml of 50 mM NiSO₄ and 15 ml of binding buffer + urea to 6 M. The soluble fraction was passed over the resin and protein that bound nonspecifically was washed from the resin with 50 ml of binding buffer followed by 50 ml of washing buffer (20 mM Tris-HCl pH 7.9, 0.5 M NaCl, 60 mM imidazole) + urea to 6 M. The HisTag-Ipa fusion protein was then eluted from the column with elution buffer (20 mM Tris-HCl pH7.9, 0.5 M NaCl, 1 M imidazole) + urea to 6 M. At each step of the purification process, the concentration of protein in the sample was determined using the bicinchoninic acid (BCA) micro-assay (Sigma Chemical Co., St. Louis, MO) according to the manufacturer's instructions. The samples were stored at -20°C and the HisBind resin was regenerated with 20 mM Tris-HCl pH 7.9, 0.5 M NaCl, 100 mM EDTA.

### Refolding of IpaC and SipC Proteins Prepared in Urea

Refolding of Ipa proteins prepared in urea was facilitated by stepwise removal of urea by dialysis until the minimum concentration of urea which did not allow precipitation of the protein sample was reached. For IpaC and SipC, this concentration was 1 to 2 M urea. Unfortunately, the gradual removal of urea at protein concentrations greater than about 0.2 to 0.3 mg/ml results in the formation of folding intermediates that are susceptible to aggregation. This problem was overcome by rapidly diluting the partially refolded protein in urea-free buffer which allowed intraprotein associations to occur between newly formed secondary structures rather than nonproductive interprotein interactions. IpaC and SipC used in fluorescence analysis of protein-protein interactions were renatured in this manner directly in the cuvette used for fluorescence measurements.

### SDS-PAGE and Western blot analysis.

SDS-PAGE was performed using the standard procedure of Laemmli, *Nature* 227:680, 1970. Following electrophoresis on 9% polyacrylamide gels, the samples could be stained with Coomassie brilliant blue R250 or the proteins electroblotted to PVDF membranes (MSI, Westborough, MA) for Western blot analysis using a BioRad Transblot Semi-dry Blotter according to the manufacturer's instructions. Western blot analysis was performed. Briefly, the membranes were blocked following protein transfer by incubation in nonfat dry milk in TBS (10 mM Tris-HCl pH 7.5, 150 mM NaCl) and then incubated with anti-SipC polyclonal antibodies or anti-IpaC monoclonal antibodies diluted in TBS containing 1 mM EDTA and 1% NP-40 (ν/ν). After several rinses in the same buffer, the membrane was incubated in ¹²⁵I-labeled protein G (100,000 dpm/ml) in the same buffer. The membrane was then rinsed in TBS containing 1 mM EDTA, 1 M NaCl and 0.4% N-laurylsarcosine (*w*/*ν*), wrapped in plastic wrap, and exposed to Fuji X-ray medical film.

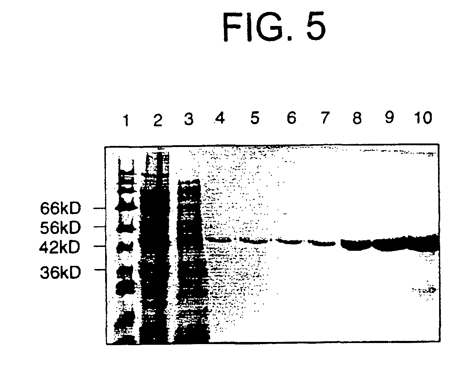

Recombinant SipC and IpaC were purified to greater than 95% homogeneity in a single step using this procedure (Figure 5). In each case, the purified protein can be seen as the predominant protein band by SDS-PAGE with Coomassie staining. In some cases, stable degradation products were also visible; however, these products (particularly for IpaC) are also observed as significant products found in a concentrated water-extract of fully virulent *S*. *flexneri* 2a. In Western blots, the recombinant SipC and IpaC proteins reacted readily with monoclonal antibodies (1H4 and 2G2, respectively) known to recognize the natural form of these proteins.

The recombinant SipC and IpaC fusion proteins described here contain a thrombin cleavage site at the junction between the target protein and its N-terminal HisTag leader. IpaC and SipC do not contain a thrombin cleavage site. Site-specific cleavage of each protein with thrombin yields a native IpaC or SipC protein product with two additional amino acids at its N terminus. After thrombin cleavage, the HisTag-containing leader peptide could be separated from the recombinant Ipa protein product by adding charged HisBind resin to the mixture and lightly centrifuging to pellet the resin (along with the HisTag leader) while leaving the soluble Ipa protein in the supernatant. Thrombin cleavage efficiency approached completion using an overnight incubation at 20°C.

### EXAMPLE 2

### Demonstration of the Ability of SipC and IpaC to enhance Immunoresponse to Ovalbumin and LPS in Mice

The ability of IpaC and SipC to act as an adjuvant was evaluated using two different antigens (ovalbumin and lipopolysaccharide ( LPS)) which do not produce a vigorous antibody response when given alone at a mucosal site.

Groups of 5 mice were immunized intranasally with 5 µg of either IpaC, SipC, or CT adjuvants alone or mixed with 10 µg of ovalbumin or LPS. Control animals received intranasal doses (10 µg) of either ovalbumin or LPS alone. Additional control animals received adjuvant alone (either IpaC, SipC, or CT). Cholera toxin (CT, Bema Scientific, Miami, FL) was used as a positive adjuvant control. A total volume of 25 µl was used for immunization doses. Prior to intranasal immunization mice were anesthetized with ketamine / rompun. The 25 µl dose was delivered in 5 to 6 small drops applied to the external nares with a micropipet. Mice were immunized on days 0, 14, and 28. Blood was taken by tail bleed from all mice on days 0, 21, and 35.

Serum antibody levels were measured by ELISA using purified ovalbumin or LPS as the antigen. Goat anti-mouse IgG or IgA labeled with alkaline phosphatase were used as conjugates.

### Results

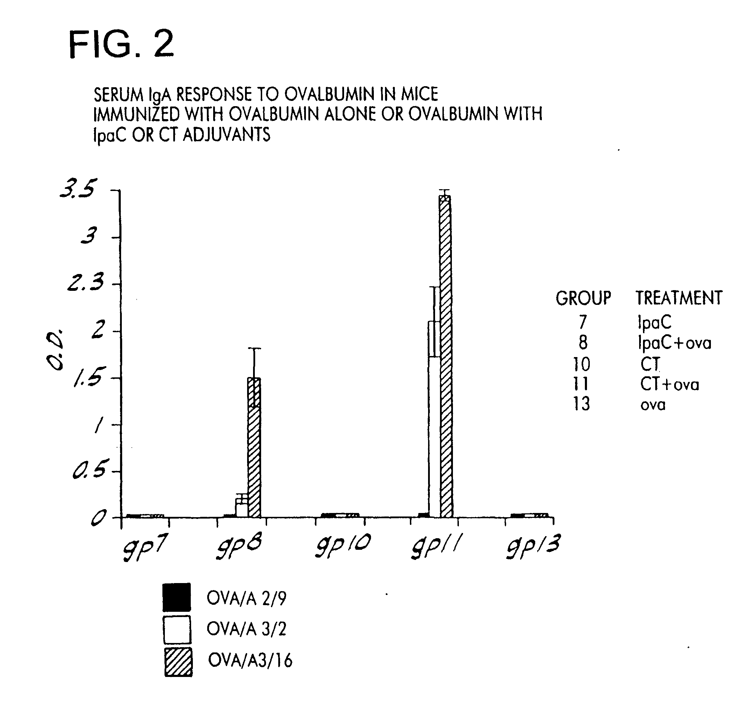

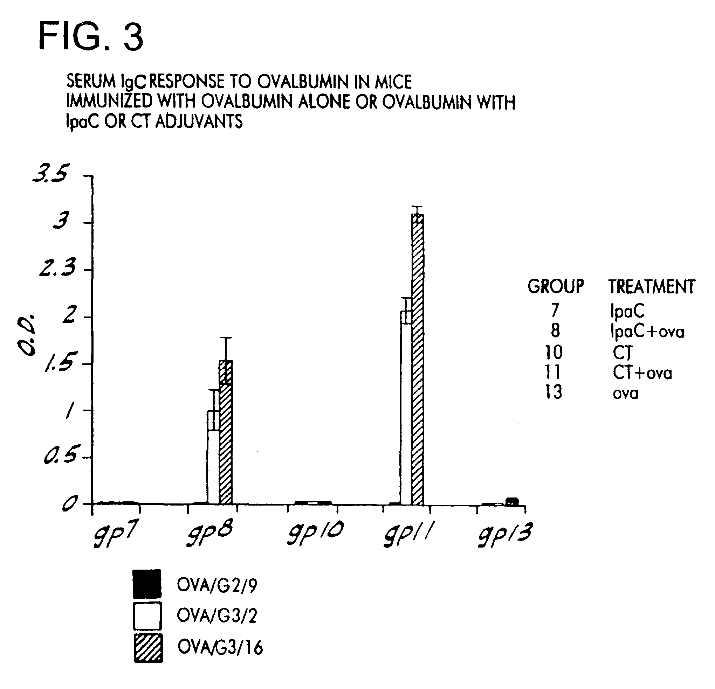

Figures 2 and 3 show that mice immunized intranasally with ovalbumin alone (group 13) do not produce a detectable serum IgA (Fig. 2) or IgG (Fig. 3) response after 2 or 3 doses of antigen. Mice immunized with an IpaC plus ovalbumin mixture (group 8) produced a pronounced serum IgA (Fig. 2) and IgG (Fig. 3) response against ovalbumin. The antibody response was comparable to a cholera toxin (a proven adjuvant) plus ovalbumin mixture (group 11). IpaC (group 7) or CT (Group 10) alone did not stimulate an anti-ovalbumin response.
Administration of purified IpaC or SipC caused no visible distress in mice. In contrast, intranasal immunization with CT led to visible lethargy and fur ruffling with eventual full recovery by the mice.

Co-administration of the purified *S*. *sonnei* LPS (10 µg) with IpaC (group 9) or SipC (group 15) produced a strong serum IgA response to LPS (see Figure 5). This result was comparable to that produced by CT plus LPS (group 12). LPS by itself did not produce an IgA response.

Thus, IpaC and SipC have strong adjuvant properties capable of stimulating both an IgG and IgA response. The adjuvant effect of IpaC and SipC is as good as or better than CT. Furthermore neither IpaC or SipC exhibited any toxicity.

### EXAMPLE 3

### Preparation of Truncated Recombinant Invasin Proteins

Several constructs containing portions of *IpaC* coding DNA sequence were prepared from the IpaC-pET15b vector construct of Example 1. *IpaC* contains the internal restriction sites StuI and NsiI which (in combination with the pBT15b cleavage sites NdeI and BamHI) allow for the removal of the N-terminal 44% of the molecule (fragment A), middle 39% of the molecule (fragment B), and C-terminal 17% of the molecule (fragment C) without needing to create new restriction sites by mutagenesis. Mutagenesis, according to any standard method, can be used to create further restriction sites. In all cases, the HisTag leader sequence is retained for affinity purification by the method outlined in Example 1.

### (HisTag-B-C)

Removal of the N-terminal 44% (about 168 amino acids) of IpaC is accomplished by treatment with NdeI and StuI which results in the removal of the epitope region I along with about 75% of the central hydrophobic portion of the molecule. Epitope regions II and III are not touched because fragments B and C remain intact with the HisTag leader. The plasmid is then ligated.

### (HisTag-A-C)

Removal of the central 39% (about 150 amino acids) of IpaC by treatment with StuI and NsiI results in removal of about 25% of the central hydrophobic region and complete removal of epitope region II. In this case, fragments A and C are ligated in-frame, and the HisTag leader is retained at the beginning of the protein.

### (HisTag-A-B)

Removal of the C-terminal 17% (about 63 amino acids) ofIpaC by treatment with NsiI and BamHI results in removal of epitope region III but does not involve any of the other characteristic regions of the molecule. Here, fragments A and B remain together with the HisTag leader. The plasmid is then ligated.

Alternatively, each fragment can be expressed with the HisTag leader but without the other two IpaC fragments (giving HisTag-A, HisTag-B, or HisTag-C). In order to retain fragment B, two restriction-ligation cycles are required: the first with NdeI and StuI to remove fragment A, and a second with BamHI and NsiI to remove fragment C.

The new constructs are then transformed into *E. coli* XL1B, cultured, and the plasmid purified and transformed into *E. coli* BL21(DE3). The transformed bacteria are then cultured, induced, and the protein purified as in Example 1. The fragments are then tested for adjuvant activity as in Example 2, with recombinant HisTag-IpaC used as a control adjuvant.

### Example 4

### Production and Demonstration of Biological Activity of IpaC PCR Deletion Mutants

### Production of IpaCΔI

IpaCΔI was produced by amplification of a 909 nucleotide sequence from the *Shigella flexneri* 2a virulence plasmid (Venkatessan et al., *Proc. Natl. Acad. Sci. USA,* 85:9317-9321, 1988) using a 5' primer consisting of GAGA (SEQ ID NO: 3), the NdeI restriction endonuclease site, and 19 bases specific to *IpaC* beginning at base 241 (based on the sequence published by Venkatesan et al., *Proc. Natl. Acad. Sci. USA,* 85:9317-9321, 1988) (GAGACATATGTTATCAGAGCAGGTTCAGC)(SEQ ID NO: 4) and a 3'primer consisting of GAGA (SEQ ID NO: 3), the BamHI restriction endonuclease site, and 20 bases specific to the 3' end of *ipaC* (GAGAGGATCCTTAAGCTCGAATGTTACCAG)(SEQ ID NO 5). The amplified sequence was ligated into pCR2.1-TOPO (Invitrogen, San Diego, CA) and transformed into *E. coli* TOP10 (Invitrogen). The plasmid was purified and the NdeI/BamHI *ipaC* fragment excised for ligation into NdeI/BamHI-digested pET15b (Novagen). This plasmid was transformed into *E. coli* NovaBlue (Invitrogen). The plasmid was then purified and transformed into *E. coli* BL21(DE3)pLysS (Novagen). The transformed bacteria were cultured, induced and the protein purified as in Example 1. The resulting protein product (IpaCΔI) was comprised of amino acids 62 to 363 of IpaC (according to the amino acid numbering system of Turbyfill et al., *Infect. Immun*., 63:3927-3935, 1995). The IpaCΔI protein was tested for biological activity as described below.

### Production of IpaCΔIII

*IpaCΔIII* was produced by amplifying a 837 nucleotide sequence from the *S*. *flexneri* virulence plasmid using a 5' primer consisting of GAGA (SEQ ID NO: 3), the NdeI restriction endonuclease site, and 21 bases specific to the 5' end of *ipaC* (GAGACATATGTTGCAAAAGCAATTTGC) (SEQ ID NO: 6) and a 3' primer consisting of GAGA (SEQ ID NO: 3), the BamHI restriction endonuclease site and 19 bases specific to *ipaC* beginning at base 837 (Venkatesan et al., *Proc. Natl. Acad. Sci. USA,* 85:9317-9321, 1988) and a translation termination site (GAGAGGATCCTTAGGTGTCAATTTTATCCTGC) (SEQ ID NO: 7). The amplified sequence was ligated into pCR2.1-TOPO and transformed into the *E. coli* TOP10. The plasmid was purified and the NdeI/BamHI fragment excised for ligation into NdeI/BamHI-digested pET15b. The plasmid was transformed into E. *coli* NovaBlue. The plasmid was then purified and transformed into *E*. *coli* BL21(DE3)pLysS. The transformed bacteria were cultured, induced and the protein purified as in Example 1. The resulting protein product (IpaCΔIII) was comprised of amino acids -19 to 260 of IpaC (Turbyfill et al., *Infect. Immun.,* 63:3927-3935, 1995). The IpaCΔIII protein was tested for biological activity as described below.

### Production of IpaCΔI/III

*IpaCΔ*I/III was produced by amplifying a 597 nucleotide sequence from the S. *flexneri* virulence plasmid using a 5' primer consisting of GAGA (SEQ ID NO: 3), the NdeI restriction endonuclease site and 19 bases specific to *ipaC* beginning at base 184 (241) (GAGACATATGTTATCAGAGCAGGTTCAGC) (SEQ ID NO: 8) and a 3' primer consisting of GAGA (SEQ ID NO: 3), the BamHI restriction endonuclease site and 19 bases specific to *ipaC* beginning at base 837 (Venkatesan et al., *Proc. Natl. Acad. Sci. USA,* 85:9317-9321, 1988) and a translation termination site (GAGAGGATCCTTAGGTGTCAATTTTATCCTGC) (SEQ ID NO: 9). The amplified sequence was ligated into pCR2.1-TOPO and transformed into *E. coli* TOP10. The plasmid was purified and the NdeI/BamHI *ipaC* fragment excised and ligated into NdeI/BamHI-digested pET15b. The ligated product was transformed into the *E. coli* NovaBlue. The plasmid was then purified and transformed into the *E. coli* BL21(DE3)pLysS. The transformed bacteria were then cultured, induced and the protein purified as described in Example 1. The resulting protein product (IpaCΔI/III was comprised of amino acids 62 to 260 of IpaC (Turbyfill et al., *Infect. Immun*., 63:3927-3935, 1995). The protein was then tested for biological activity as described below.

### Production of IpaCΔII

*IpaCΔII* was produced by amplifying a 585 nucleotide sequence from the *S*. *flexneri* virulence plasmid using a 5' primer consisting of GAGA (SEQ ID NO: 3), the NdeI restriction endonuclease site, and 21 bases specific to the 5' end of *ipaC* (GAGACATATGTTGCAAAAGCAA) (SEQ ID NO: 10) and a 3'primer consisting of GAGA (SEQ ID NO: 3), the XhoI restriction endonuclease site and 19 bases specific to *ipaC* beginning at base 585 (Venkatesan et al., *Proc. Natl. Acad. Sci. USA,* 85:9317-9321, 1988) (GAGACTCGAGATGCGTTTTTTTGGCACCG) (SEQ ID NO: 11). The amplified sequence was ligated into pCR2.1-TOPO and transformed into *E. coli* TOP10. A 315 nucleotide sequence was then amplified using a 5' primer consisting of GAGA (SEQ ID NO: 3), a XhoI restriction endonuclease site and 19 bases specific to *ipaC* beginning at base 834 (Venkatesan et al. 1988) (GAGACTCGAGACCCAGAGAAGAACTTACG) (SEQ ID NO: 12) and a 3' primer consisting of GAGA (SEQ ID NO: 3), the BamHI restriction endonuclease site and 20 bases specific to the 3' end of *ipaC* (GAGAGGATCCTTAAGCTCGAATGTTACCAG) (SEQ ID NO: 13). The amplified sequence was ligated into pCR2.1-TOPO and transformed into E. *coli* TOP10. Each plasmid was purified, the respective NdeI/XhoI and XhoI/BamHI *ipaC* fragments excised and the two ligated together. This two-part fragment was then ligated into NdeI/BamHI-digested pET15b. The ligated product was transformed into the *E. coli* NovaBlue. The plasmid was purified and transformed into *E. coli* BL21(DE3)pLysS. The transformed bacteria were then cultured, induced and the protein purified as in Example1. The resulting protein product (IpaCΔII) comprised amino acids -19 to 176 and 261 to 363 ofIpaC (Turbyfill et al., *Infect. Immun.,* 63:3927-3935, 1995). The protein was tested for biological activity as described below.

### Production of IpaCΔH

*IpaCΔH* was produced by amplifying a 243 nucleotide sequence from the *S. flexneri* virulence plasmid using a 5' primer consisting of GAGA (SEQ ID NO: 3), the NdeI restriction endonuclease site, and 21 bases specific to the 5' end of *ipaC* (GAGACATATGTTGCAAAAGCAATTTGC) (SEQ ID NO: 14) and a 3' primer consisting of GAGA (SEQ ID NO: 3), the XhoI restriction endonuclease site, and 21 bases specific to *ipaC* beginning at base 243 (Ventakesan et al., *Proc. Natl. Acad. Sci. USA,* 85:9317-9321, 1988) (GAGACTCGAGTAACTTTAAAAGTTGATCATC) (SEQ ID NO: 15). The amplified sequence was ligated into pCR2.1-TOPO and transformed into the *E. coli* TOP10. A 315- nucleotide sequence was amplified using a 5' primer consisting of GAGA (SEQ ID NO: 3), a XhoI restriction endonuclease site and 18 bases specific to *ipaC* beginning at base 624 (Venkatesan et al., *Proc. Natl. Acad. Sci. USA,* 85:9317-9321, 1988) (GAGACTCGAGCTTGCCACTGCTCAATCT) (SEQ ID NO: 16) and a 3' primer consisting of GAGA (SEQ ID NO: 3), the BamHI restriction endonuclease site and 20 bases specific to the 3' end of *ipaC* (GAGAGGATCCTTAAGCTCGAATGTTACCAG) (SEQ ID NO: 17). The amplified sequence was ligated into pCR2.1-TOPO and transformed into the E. *coli* TOP10. Each plasmid was purified, and the respective NdeI/XhoI and XhoI/BamHI *ipaC* fragments were excised and ligated to each other. This two-part fragment was then ligated into NdeI/BamHI digested pET15b. The ligated product was transformed into *E. coli* NovaBlue. The plasmid was purified and transformed into *E*. *coli* BL21(DE3)pLysS. The transformed bacteria were then cultured, induced and the protein purified as described in Example 1. The resulting protein product (IpaCΔH) was comprised of amino acids -19 to 62 and 189 to 363 of IpaC (Turbyfill et al., *Infect. Immun*., 63:3927-3935, 1995). The protein was tested for biological activity as described below.

### Determination of Biological Activity Based on Stimulation of S. flexneri Uptake

The biological activity of IpaC deletion mutant proteins was determined by measuring the ability of the protein to increase uptake of *S. flexneri* by cultured epithelial cells. Methods for measuring uptake of *S. flexneri* are known in the art (Niesel et al., *J. Clin. Microbiol.,* 22:897-902, 1985; Marquart et al., *Infect Immunol.,* 64:4182-4187, 1996). Briefly, Henle 407 cells were grown to near confluence in 24-well tissue culture plates. *S. flexneri* 2a grown to an A₆₀₀ of about 0.4 was diluted with MEME containing 0.67 ug of FeCl₃ per ml and 0.45% glucose (MEME-Fe) so that a final multiplicity of infection (MOI) of about 3 was reached. To each well containing Henle 707 cells MEME-Fe was added alone (control) or with 0.1 to 1 µM of the protein to be tested for biological activity. Immediately after, *S. flexneri* organisms were added and incubated with the cells to 30 minutes at 37°C. Following incubation, the bacterial suspension was removed by aspiration, and the cells washed six times (with a 1 minute incubation for each wash) with MEME containing 5% newborn calf serum and 40 µg of gentamicin per ml. The cells were then incubated with a final gentamicin-containing wash for 2 hours. This treatment kills any bacteria remaining exposed to the medium, but not those bacteria that have been internalized. The cells were next washed with MEME-Fe lacking serum and gentamicin. Each epithelial cell monolayer was then overlaid with 0.5% agar containing 2X Luria-Bertain medium. Each plate was then incubated at room temperature for 30 minute and then inverted for incubation at 37°C overnight. The *S. flexneri* organisms protected from gentamicin due to uptake by the Henle 407 cells are seen as subsurface colonies and can be counted by any suitable method, for example, using a dissecting microscope.

### Results

Addition of IpaCΔII protein to culture of Henle 407 cells enhanced uptake of *S*. *flexneri* by 220% as compared to controls to which no IpaCΔII protein was added. These results show that the IpaCΔII mutant protein retains its biological activity.

### EXAMPLE 5

### Characterization of Immune Response to Ovalbumin in Mice Immunized Using Purified Recombinant Shigella IpaC Adjuvant

### Immunization of Mice with IpaC or Cholera Toxin

Groups of 5 Balb/c mice were immunized intranasally three times with 5 µg ofIpaC or cholera toxin (CT) adjuvants alone or mixed with 10 µg of ovalbumin (OVA). Control animals received intranasal doses of 10 µg of either ovalbumin alone or adjuvants alone. A total volume of 25 µl was used for each immunization dose. Prior to intranasal immunization, mice were anesthetized with ketamine/rompun. The 25 µl dose was delivered in 5 to 6 small drops applied to the external nares with a micropipet. Mice were immunized on days 0, 14 and 28. Blood was taken by tail bleed from all mice on days 0, 28 and 42.

### ELISA Assay

An ELISA assay was used to measure the levels of IgG subclasses to ovalbumin following immunization. The amount of ovalbumin used in the assay to coat the assay wells was 1 µg/well. Primary antibodies from the blood samples obtained are diluted 1:360 in 2% casein and are incubated with the ovalbumin antigen for 4 hours. After washing in PBS/Tween 20, plates were probed for 1 hour with monoclonal antibodies against mouse IgG subclasses IgG1, IgG2a, IgG2b, and IgG3 labeled with alkaline phosphatase obtained from Pharmingen, Inc., San Diego, CA. The optical density (O.D.) was measured at 405 nm.

### Results

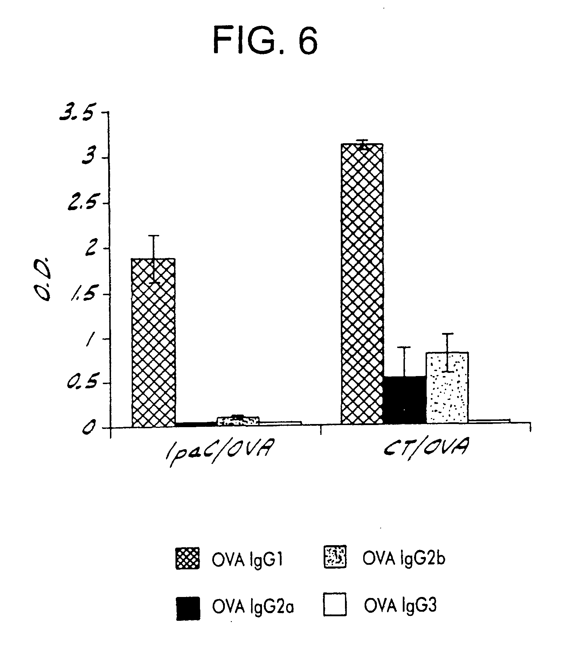

Figure 6 shows that the predominant anti-ovalbumin IgG subclass produced in mice immunized with CT mixed with ovalbumin was IgG1. This result is in agreement with previous work using CT adjuvants. IgG1 was also the predominant IgG subclass in serum of mice immunized with IpaC mixed with ovalbumin. Low levels of IgG2b were also produced in mice immunized with either IpaC or CT mixed with ovalbumin. This pattern of IgG subclasses indicates an IL4/Th2 driven response characteristic of a mucosal immune response.

In light of the detailed description of the invention and the examples presented above, it can be appreciated that the several aspects of the invention are achieved.

It is to be understood that the present invention has been described in detail by way of illustration and example in order to acquaint others skilled in the art with the invention, its principles, and its practical application. Particular formulations and processes of the present invention are not limited to the descriptions of the specific embodiments presented, but rather the descriptions and examples should be viewed in terms of the claims that follow and their equivalents. While some of the examples and descriptions above include some conclusions about the way the invention may function, the inventors do not intend to be bound by those conclusions and functions, but put them forth only as possible explanations.

It is to be further understood that the specific embodiments of the present invention as set forth are not intended as being exhaustive or limiting of the invention, and that many alternatives, modifications, and variations will be apparent to those of ordinary skill in the art in light of the foregoing examples and detailed description. Accordingly, this invention is intended to embrace all such alternatives, modifications, and variations that fall within the spirit and scope of the following claims.

### SEQUENCE LISTING

<151> 1999-06-01
<160> 17
<170> PatentIn Ver. 2.0
<210> 1
   <211> 409
   <212> PRT
   <213> Salmonella typhimurium
<400> 1
   Met Leu Ile Ser Asn Val Gly Ile Asn Pro Ala Ala Tyr Leu Asn Asn
   1 5 10 15
   His Ser Val Glu Asn Ser Ser Gln Thr Ala Ser Gln Ser Val Ser Ala
   20 25 30
   Lys Asp Ile Leu Asn Ser Ile Gly Ile Ser Ser Ser Lys Val Ser Asp
   35 40 45
   Leu Gly Leu Ser Pro Thr Leu Ser Ala Pro Ala Pro Gly Val Leu Thr
   50 55 60
   Gln Thr Pro Gly Thr Ile Thr Ser Ser Leu Lys Ala Ser Ile Gln Asn
   65 70 75 80
   Thr Asp Met Asn Gln Asp Leu Asn Ala Leu Ala Asn Asn Val Thr Thr
   85 90 95
   Lys Ala Asn Glu Val Val Gln Thr Gln Leu Arg Glu Gln Gln Ala Glu
   100 105 110
   Val Gly Lys Phe Phe Asp Ile Ser Gly Met Ser Ser Ser Ala Val Ala
   115 120 125
   Leu Leu Ala Ala Ala Asn Thr Leu Met Leu Thr Leu Asn Gln Ala Asp
   130 135 140
   Ser Lys Leu Ser Gly Lys Leu Ser Leu Val Ser Phe Asp Ala Ala Lys
   145 150 155 160
   Thr Thr Ala Ser Ser Met Met Arg Glu Gly Met Asn Ala Leu Ser Gly
   165 170 175
   Ser Ile Ser Gln Ser Ala Leu Gln Leu Gly Ile Thr Gly Val Gly Ala
   180 185 190
   Lys Leu Glu Tyr Lys Gly Leu Gln Asn Glu Arg Gly Ala Leu Lys His
   195 200 205
   Asn Ala Ala Lys Ile Asp Lys Leu Thr Thr Glu Ser His Ser Ile Lys
   210 215 220
   Asn Val Leu Asn Gly Gln Asn Ser Val Lys Leu Gly Ala Glu Gly Val
   225 230 235 240
   Asp Ser Leu Lys Ser Leu Asn Ile Arg Lys Pro Val Pro Met Arg Arg
   245 250 255
   Lys Ile Leu Met Met Arg Arg Leu Asn Leu Met Pro Glu Pro Ala Pro
   260 265 270
   Arg Lys Val Trp Val Leu Lys Thr Val Ile Asn Lys Val Ser Leu Asn
   275 280 285
   Ile Tyr Ile Leu Ser Lys Arg Leu Glu Ser Val Glu Ser Asp Ile Arg
   290 295 300
   Leu Glu Gln Asn Tyr Met Asp Ile Thr Arg Ile Asp Ser Ala Gln Asp
   305 310 315 320
   Ala Asp Asp Gly Arg Ser Asp Tyr Glu Glu Leu Gly His Gly Arg Trp
   325 330 335
   Tyr Cys Arg Gly Val Arg Ala Val Arg Arg Tyr Ser Gly Asn Val Ser
   340 345 350
   Glu Gln Gln Ile Ser Gln Val Asn Asn Arg Val Ala Ser Thr Ala Ser
   355 360 365
   Asp Glu Ala Arg Glu Ser Ser Arg Lys Ser Thr Ser Leu Ile Gln Glu
   370 375 380
   Met Leu Lys Thr Met Glu Ser Ile Asn Gln Ser Lys Ala Ser Ala Leu
   385 390 395 400
   Ala Ala Ile Ala Gly Asn Ile Arg Ala
   405
<210> 2
   <211> 382
   <212> PRT
   <213> Shigella flexneri
<400> 2
   Met Leu Gln Lys Gln Phe Cys Asn Lys Leu Leu Leu Asp Thr Asn Lys
   1 5 10 15
   Glu Asn Val Met Glu Ile Gln Asn Thr Lys Pro Thr Gln Thr Leu Tyr
   20 25 30
   Thr Asp Ile Ser Thr Lys Gln Thr Gln Ser Ser Ser Glu Thr Gln Lys
   35 40 45
   Ser Gln Asn Tyr Gln Gln Ile Ala Ala His Ile Pro Leu Asn Val Gly
   50 55 60
   Lys Asn Pro Val Leu Thr Thr Thr Leu Asn Asp Asp Gln Leu Leu Lys
   65 70 75 80
   Leu Ser Glu Gln Val Gln His Asp Ser Glu Ile Ile Ala Arg Leu Thr
   85 90 95
   Asp Lys Lys Met Lys Asp Leu Ser Glu Met Ser His Thr Leu Thr Pro
   100 105 110
   Glu Asn Thr Leu Asp Ile Ser Ser Leu Ser Ser Asn Ala Val Ser Leu
   115 120 125
   Ile Ile Ser Val Ala Val Leu Leu Ser Ala Leu Arg Thr Ala Glu Thr
   130 135 140
   Lys Leu Gly Ser Gln Leu Ser Leu Ile Ala Phe Asp Ala Thr Lys Ser
   145 150 155 160
   Ala Ala Glu Asn Ile Val Arg Gln Gly Leu Ala Ala Leu Ser Ser Ser
   165 170 175
   Ile Thr Gly Ala Val Thr Gln Val Gly Ile Thr Gly Ile Gly Ala Lys
   180 185 190
   Lys Thr His Ser Gly Ile Ser Asp Gln Lys Gly Ala Leu Arg Lys Asn
   195 200 205
   Leu Ala Thr Ala Gln Ser Leu Glu Lys Glu Leu Ala Gly Ser Lys Leu
   210 215 220
   Gly Leu Asn Lys Gln Ile Asp Thr Asn Ile Thr Ser Pro Gln Thr Asn
   225 230 235 240
   Ser Ser Thr Lys Phe Leu Gly Lys Asn Lys Leu Ala Pro Asp Asn Ile
   245 250 255
   Ser Leu Ser Thr Glu His Lys Thr Ser Leu Ser Ser Pro Asp Ile Ser
   260 265 270
   Leu Gln Asp Lys Ile Asp Thr Gln Arg Arg Thr Tyr Glu Leu Asn Thr
   275 280 285
   Leu Ser Ala Gln Gln Lys Gln Asn Ile Gly Arg Ala Thr Met Glu Thr
   290 295 300
   Ser Ala Val Ala Gly Asn Ile Ser Thr Ser Gly Gly Arg Tyr Ala Ser
   305 310 315 320
   Ala Leu Glu Glu Glu Glu Gln Leu Ile Ser Gln Ala Ser Ser Lys Gln
   325 330 335
   Ala Glu Glu Ala Ser Gln Val Ser Lys Glu Ala Ser Gln Ala Thr Asn
   340 345 350
   Gln Leu Ile Gln Lys Leu Leu Asn Ile Ile Asp Ser Ile Asn Gln Ser
   355 360 365
   Lys Asn Ser Ala Ala Ser Gln Ile Ala Gly Asn Ile Arg Ala
   370 375 380
<210> 3
   <211> 4
   <212> DNA
   <213> Artificial Sequence
<220>
   <223> Description of Artificial Sequence: NdeI restrictioni site
<400> 3
   gaga 4
<210> 4
   <211> 29
   <212> DNA
   <213> Artificial Sequence
<220>
   <223> Description of Artificial Sequence: PCR Primer
<400> 4
   gagacatatg ttatcagagc aggttcagc 29
<210> 5
   <211> 30
   <212> DNA
   <213> Artificial Sequence
<220>
   <223> Description of Artificial Sequence: PCR Primer
<400> 5
   gagaggatcc ttaagctcga atgttaccag 30
<210> 6
   <211> 27
   <212> DNA
   <213> Artificial Sequence
<220>
   <223> Description of Artificial Sequence: PCR Primer
<400> 6
   gagacatatg ttgcaaaagc aatttgc 27
<210> 7
   <211> 32
   <212> DNA
   <213> Artificial Sequence
<220>
   <223> Description of Artificial Sequence: PCR Primer
<400> 7
   gagaggatcc ttaggtgtca attttatcct gc 32
<210> 8
   <211> 29
   <212> DNA
   <213> Artificial Sequence
<220>
   <223> Description of Artificial Sequence: PCR Primer
<400> 8
   gagacatatg ttatcagagc aggttcagc 29
<210> 9
   <211> 32
   <212> DNA
   <213> Artificial Sequence
<220>
   <223> Description of Artificial Sequence: PCR Primer
<400> 9
   gagaggatcc ttaggtgtca attttatcct gc 32
<210> 10
   <211> 22
   <212> DNA
   <213> Artificial Sequence
<220>
   <223> Description of Artificial Sequence: PCR Primer
<400> 10
   gagacatatg ttgcaaaagc aa 22
<210> 11
   <211> 29
   <212> DNA
   <213> Artificial Sequence
<220>
   <223> Description of Artificial Sequence: PCR Primer
<400> 11
   gagactcgag atgcgttttt ttggcaccg 29
<210> 12
   <211> 29
   <212> DNA
   <213> Artificial Sequence
<220>
   <223> Description of Artificial Sequence: PCR Primer
<400> 12
   gagactcgag acccagagaa gaacttacg 29
<210> 13
   <211> 30
   <212> DNA
   <213> Artificial Sequence
<220>
   <223> Description of Artificial Sequence: PCR Primer
<400> 13
   gagaggatcc ttaagctcga atgttaccag 30
<210> 14
   <211>27
   <212> DNA
   <213> Artificial Sequence
<220>
   <223> Description of Artificial Sequence: PCR Primer
<400> 14
   gagacatatg ttgcaaaagc aatttgc 27
<210> 15
   <211> 31
   <212> DNA
   <213> Artificial Sequence
<220>
   <223> Description of Artificial Sequence: PCR Primer
<400> 15
   gagactcgag taactttaaa agttgatcat c 31
<210> 16
   <211> 28
   <212> DNA
   <213> Artificial Sequence
<220>
   <223> Description of Artificial Sequence: PCR Primer
<400> 16
   gagactcgag cttgccactg ctcaatct 28
<210> 17
   <211> 30
   <212> DNA
   <213> Artificial Sequence
<220>
   <223> Description of Artificial Sequence: PCR Primer
<400> 17
   gagaggatcc ttaagctcga atgttaccag 30

## Claims

1. The use of a purified recombinant invasin protein as adjuvant for the manufacture of a vaccine composition, whereby administration of a purified recombinant invasin composition to an animal in combination with an antigen elicits an immune response to the antigen.

2. The use according to claim 1, wherein the immune response is **characterized by** the production of at least one cytokine by Th2 cells.

3. The use according to claim 2, wherein the at least one cytokine is an interleukin (IL).

4. The use according to claim 3, wherein the interleukin (IL) is chosen from IL-4, IL-5, IL-6, IL-10 and IL-13.

5. The use according to claim 1, wherein the immune response is **characterized by** production of at least one class of immunoglobulin chosen from IgG, IgE, IgM and IgA.

6. The use according to any one of claims 1 to 5, wherein the purified recombinant invasin protein comprises an amino acid sequence derived from an invasin protein of a bacterium chosen from *Shigella spp., Salmonella* spp., and enteroinvasive *E. coli.*

7. The use according to any one of claims 1 to 6, wherein the purified recombinant invasin protein comprises an amino acid sequence of at least 15 amino acids.

8. The use according to any one of claims 1 to 7, wherein the purified recombinant invasin protein comprises an amino acid sequence in which no more than 35% of the amino acid residues have been conservatively substituted.

9. The use according to any one of claims 1 to 8, wherein the purified recombinant protein is an IpaC or a SipC protein.

10. The use according to claim 9, wherein the purified recombinant invasin protein comprises an amino acid sequence chosen from SEQ ID NO: 1 and SEQ ID NO: 2.

11. The use according to claim 9, wherein the purified recombinant invasin protein comprises a mutant selected from
(i) a protein obtainable by removal of the N-terminal 44% (about 168 amino acids) of IpaC by treatment with NdeI and StuI (HisTag-B-C);
(ii) a protein obtainable by removal of the central 39% (about 150 amino acids) of IpaC by treatment with StuI and NsiI (HisTag-A-C);
(iii) a protein obtainable by removal of the C-terminal 17% (about 63 amino acids) of IpaC by treatment with NsiI and BamHI (HisTag-A-B);
(iv) a protein comprised of amino acids 62 to 363 of IpaC (IpaCΔ1);
(v) a protein comprised of amino acids -19 to 62 and 189 to 363 of IpaC (IpaCΔH);
(vi) a protein comprised of amino acids -19 to 176 and 261 to 363 of IpaC (IpaCΔII); and
(vii) a protein comprised of amino acids -19 to 260 of IpaC (IpaCAIII).

12. The use according to any one of claims 1 to 11, wherein the purified recombinant invasin protein is of at least 95% purity.

13. The use according to any one of claims 1 to 12, wherein the purified recombinant invasin protein is at least 97% pure.

14. A vaccine preparation comprising, in addition to at least one antigen, an adjuvant composition comprising at least one purified recombinant invasin protein and a pharmaceutically acceptable carrier, diluent or excipient.

15. A vaccine preparation according to claim 14, wherein the ratio of antigen to purified recombinant invasin protein is about one part antigen to between about 0.0001 to about 10,000 parts purified invasin protein.

16. A vaccine preparation according to claim 14 or claim 15, wherein the purified recombinant invasin protein is at least 95% pure.

17. A vaccine preparation according to any one of claims 14 to 16, wherein the purified recombinant invasin protein is at least 97% pure.

18. A vaccine preparation according to any one of claims 14 to 17, wherein the purified recombinant invasin protein is derived from an organism against which immunity is desired.

19. A vaccine preparation according to any one of claims 14 to 18, wherein the purified recombinant invasin protein comprises an amino acid sequence derived from an invasin protein of a bacterium chosen from Shigella spp., *Salmonella* spp., and enteroinvasive *E. coli*.

20. A vaccine preparation according to claim 19, wherein the purified recombinant protein is an IpaC or a SipC protein.

21. A vaccine preparation according to claim 19, wherein the purified recombinant invasin protein comprises an amino acid sequence chosen from SEQ ID NO: 1 and SEQ ID NO: 2.

22. A vaccine preparation according to claim 19,
wherein the purified recombinant invasin protein comprises a mutant selected from
(i) a protein obtainable by removal of the N-terminal 44% (about 168 amino acids) of IpaC by treatment with NdeI and StuI (HisTag-B-C);
(ii) a protein obtainable by removal of the central 39% (about 150 amino acids) of IpaC by treatment with StuI and NsiI (HisTag-A-C);
(iii) a protein obtainable by removal of the C-terminal 17% (about 63 amino acids) of IpaC by treatment with NsiI and BamHI (HisTag-A-B);
(iv) a protein comprised of amino acids 62 to 363 of IpaC (IpaCΔI);
(v) a protein comprised of amino acids -19 to 62 and 189 to 363 of IpaC (IpaCΔH);
(vi) a protein comprised of amino acids -19 to 176 and 261 to 363 of IpaC (IpaCΔII); and
(vii) a protein comprised of amino acids -19 to 260 of IpaC (IpaCΔIII).

23. A vaccine preparation according to any one of claims 14 to 22, wherein the purified recombinant invasin protein comprises an amino acid sequence of at least 15 amino acids.

24. A vaccine preparation according to any one of claims 14 to 23, wherein the purified recombinant invasin protein comprises an amino acid sequence in which no more than 35% of the amino acid residues have been conservatively substituted.

25. A vaccine preparation according to any one of claims 14 to 24, wherein the antigen is chemically conjugated or genetically fused onto the recombinant invasin protein.

## Patentansprüche

1. Verwendung eines gereinigten rekombinanten Invasinproteins als Hilfsstoff für die Herstellung einer Impfstoffzusammensetzung, wobei die Verabreichung einer gereinigten rekombinanten Invasinzusammensetzung an ein Tier in Kombination mit einem Antigen eine Immunantwort auf das Antigen auslöst.

2. Verwendung nach Anspruch 1, bei der die Immunantwort durch die Bildung wenigstens eines Cytokins durch Th2-Zellen gekennzeichnet ist.

3. Verwendung nach Anspruch 2, bei der das wenigstens eine Cytokin ein Interleukin (IL) ist.

4. Verwendung nach Anspruch 3, bei der das Interleukin (IL) unter IL-4, IL-5, IL-6, IL-10 und IL-13 ausgewählt ist.

5. Verwendung nach Anspruch 1, bei der die Immunantwort durch Bildung wenigstens einer unter IgG, IgE, IgM und IgA ausgewählten Immunglobulin-Klasse gekennzeichnet ist.

6. Verwendung nach einem der Ansprüche 1 bis 5, bei der das gereinigte rekombinante Invasinprotein eine Aminosäuresequenz enthält, die von einem Invasinprotein eines Bakteriums stammt, das unter Shigella spp., Salmonella spp. und enteroinvasivem E. coli ausgewählt ist.

7. Verwendung nach einem der Ansprüche 1 bis 6, bei der das gereinigte rekombinante Invasinprotein eine Aminosäuresequenz von wenigstens 15 Aminosäuren aufweist.

8. Verwendung nach einem der Ansprüche 1 bis 7, bei der das gereinigte rekombinante Invasinprotein eine Aminosäuresequenz aufweist, in der nicht mehr als 35% der Aminosäurereste konservativ substituiert wurden.

9. Verwendung nach einem der Ansprüche 1 bis 8, bei der das gereinigte rekombinante Protein ein IpaC- oder ein SipC-Protein ist.

10. Verwendung nach Anspruch 9, bei der das gereinigte rekombinante Invasinprotein eine Aminosäuresequenz aufweist, die unter SEQ ID NO: 1 und SEQ ID NO: 2 ausgewählt ist.

11. Verwendung nach Anspruch 9, bei der das gereinigte rekombinante Invasinprotein eine Mutante aufweist, die ausgewählt ist unter
(i) einem Protein, das durch Entfernung der N-terminalen 44% (etwa 168 Aminosäuren) des IpaC durch Behandlung mit NdeI und StuI (HisTag-B-C) erhältlich ist,
(ii) einem Protein, das durch Entfernung der zentralen 39% (etwa 150 Aminosäuren) das IpaC durch Behandlung mit StuI und NsiI (HisTag-A-C) erhältlich ist,
(iii) einem Protein, das durch Entfernung der C-terminalen 17% (etwa 63 Aminosäuren) des IpaC durch Behandlung mit NsiI und BamHI (HisTag-A-B) erhältlich ist,
(iv) einem Protein aus Aminosäuren 62 bis 363 des IpaC (IpaCΔI),
(v) einem Protein aus Aminosäuren -19 bis 62 und 189 bis 363 des IpaC (IpaCΔH),
(vi) einem Protein aus Aminosäuren -19 bis 176 und 261 bis 363 des IpaC (IpaCΔII) und
(vii) einem Protein aus Aminosäuren -19 bis 260 des IpaC (IpaCΔIII).

12. Verwendung nach einem der Ansprüche 1 bis 11, bei der das gereinigte rekombinante Invasinprotein von wenigstens 95 %iger Reinheit ist.

13. Verwendung nach einem der Ansprüche 1 bis 12, bei der das gereinigte rekombinante Invasinprotein wenigstens 97% rein ist.

14. Impfstoffpräparat, das neben wenigstens einem Antigen eine Hilfsstoffzusammensetzung mit wenigstens einem gereinigten rekombinanten Invasinprotein und einem pharmazeutisch zulässigen Träger, Verdünnungsmittel oder Füllstoff enthält.

15. Impfstoffpräparat nach Anspruch 14, bei dem das Verhältnis von Antigen zu gereinigtem rekombinantem Invasinprotein etwa ein Teil Antigen zu etwa 0,0001 bis etwa 10.000 Teilen gereinigtem Invasinprotein beträgt.

16. Impfstoffpräparat nach Anspruch 14 oder Anspruch 15, bei dem das gereinigte rekombinante Invasinprotein wenigstens 95 % rein ist.

17. Impfstoffpräparat nach einem der Ansprüche 14 bis 16, bei dem das gereinigte rekombinante Invasinprotein wenigstens 97 % rein ist.

18. Impfstoffpräparat nach einem der Ansprüche 14 bis 17, bei dem das gereinigte rekombinante Invasinprotein aus einem Organismus stammt, gegen den Immunität erwünscht ist.

19. Impfstoffpräparat nach einem der Ansprüche 14 bis 18, bei dem das gereinigte rekombinante Invasinprotein eine Aminosäuresequenz enthält, die von einem Invasinprotein eines Bakteriums stammt, das unter Shigella spp., Salmonella spp. und enteroinvasivem E. coli ausgewählt ist.

20. Impfstoffpräparat nach Anspruch 19, bei dem das gereinigte rekombinante Protein ein IpaC- oder ein SipC-Protein ist.

21. Impfstoffpräparat nach Anspruch 19, bei dem das gereinigte rekombinante Invasinprotein eine Aminosäuresequenz aufweist, die unter SEQ ID NO: 1 und SEQ ID NO: 2 ausgewählt ist.

22. Impfstoffpräparat nach Anspruch 19, bei dem das gereinigte rekombinante Invasinprotein eine Mutante aufweist, die ausgewählt ist unter
(i) einem Protein, das durch Entfernung der N-terminalen 44% (etwa 168 Aminosäuren) des IpaC durch Behandlung mit NdeI und StuI (HisTag-B-C) erhältlich ist,
(ii) einem Protein, das durch Entfernung der zentralen 39% (etwa 150 Aminosäuren) das IpaC durch Behandlung mit StuI und NsiI (HisTag-A-C) erhältlich ist,
(iii) einem Protein, das durch Entfernung der C-terminalen 17% (etwa 63 Aminosäuren) des IpaC durch Behandlung mit NsiI und BamHI (HisTag-A-B) erhältlich ist,
(iv) einem Protein aus Aminosäuren 62 bis 363 des IpaC (IpaCΔI),
(v) einem Protein aus Aminosäuren -19 bis 62 und 189 bis 363 des IpaC (IpaCΔH),
(vi) einem Protein aus Aminosäuren -19 bis 176 und 261 bis 363 des IpaC (IpaCΔII) und
(vii) einem Protein aus Aminosäuren -19 bis 260 des IpaC (IpaCΔIII).

23. Impfstoffpräparat nach einem der Ansprüche 14 bis 22, bei dem das gereinigte rekombinante Invasinprotein eine Aminosäuresequenz von wenigstens 15 Aminosäuren aufweist.

24. Impfstoffpräparat nach einem der Ansprüche 14 bis 23, bei dem das gereinigte rekombinante Invasinpräparat eine Aminosäuresequenz aufweist, in der nicht mehr als 35 % der Aminosäurereste konservativ substituiert wurden.

25. Impfstoffpräparat nach einem der Ansprüche 14 bis 24, bei dem das Antigen mit dem rekombinanten Invasinprotein chemisch verbunden oder genetisch verschmolzen ist.

## Revendications

1. Utilisation d'une protéine invasine recombinante purifiée en tant qu'adjuvant pour la fabrication d'une composition de vaccin, par laquelle une administration d'une combinaison d'invasine recombinante purifiée à un animal en combinaison avec un antigène induit une réponse immunitaire contre l'antigène.

2. Utilisation conforme à la revendication 1, dans laquelle la réponse immunitaire est **caractérisée par** la production d'au moins une cytokine par des cellules Th2.

3. Utilisation conforme à la revendication 2, dans laquelle la au moins une cytokine est une interleukine (IL).

4. Utilisation conforme à la revendication 3, dans laquelle l'interleukine (IL) est choisie parmi IL-4, IL-5, IL-6, IL-10 et IL-13.

5. Utilisation conforme à la revendication 1, dans laquelle la réponse immunitaire est **caractérisée par** la production d'au moins une classe d'immunoglobuline choisie parmi IgG, IgE, IgM et IgA.

6. Utilisation conforme à n'importe laquelle des revendications 1 à 5, dans laquelle la protéine invasine recombinante purifiée comprend une séquence d'acides aminés dérivée d'une protéine invasine d'une bactérie choisie parmi *Shigella* spp., Salmonella spp. et E. coli entéroinvasif.

7. Utilisation conforme à n'importe laquelle des revendications 1 à 6, dans laquelle la protéine invasine recombinante purifiée comprend une séquence d'acides aminés d'au moins 15 acides aminés.

8. Utilisation conforme à n'importe laquelle des revendications 1 à 7, dans laquelle la protéine invasine recombinante purifiée comprend une séquence d'acides aminés dans laquelle pas plus de 35% des résidus d'acides aminés correspondent à des substitutions conservatrices.

9. Utilisation conforme à n'importe laquelle des revendications 1 à 8, dans laquelle la protéine invasine recombinante purifiée est une protéine IpaC ou une protéine SipC.

10. Utilisation conforme à la revendication 9, dans laquelle la protéine invasine recombinante purifiée comprend une séquence d'acides aminés choisie parmi la SEQ ID NO : 1 et la SEQ ID NO : 2.

11. Utilisation conforme à la revendication 9, dans laquelle la protéine invasine recombinante purifiée comprend un mutant choisi parmi
(i) une protéine pouvant être obtenue en éliminant 44% de la portion N terminale (environ 168 acides aminés) d'une IpaC par traitement avec une Ndel et une StuI (HisTag-B-C);
(ü) une protéine pouvant être obtenue en éliminant 39 % de la portion centrale (environ 150 acides aminés) d'une IpaC par traitement avec une StuI et une Nsil (HisTag-A-C);
(iii) une protéine pouvant être obtenue en éliminant 17% de la portion C terminale (environ 63 acides aminés) d'une IpaC par traitement avec une NsiI et une BamHI (HisTag-A-B) ;
(iv) une protéine constituée des acides aminés 62 à 363 d'une IpaC (IpaCΔI);
(v) une protéine constituée des acides aminés -19 à 62 et 189 à 363 d'une IpaC (ipaCΔH);
(vi) une protéine constituée des acides aminés-19 à 176 et 261 à 363 d'une IpaC (IpaCΔII); et
(vii) une protéine constituée des acides aminés -19 à 260 d'une IpaC (IpaCΔIII).

12. Utilisation conforme à n'importe laquelle des revendications 1 à 11, dans laquelle la protéine invasine recombinante purifiée possède une pureté d'au moins 95 %.

13. Utilisation conforme à n'importe laquelle des revendications 1 à 11, dans laquelle la protéine invasine recombinante purifiée est pure à au moins 97 %.

14. Préparation de vaccin comprenant, en plus d'au moins un antigène, une composition d'adjuvant comprenant au moins une protéine invasine recombinante purifiée et un véhicule pharmaceutiquement acceptable, un diluant ou un excipient.

15. Préparation de vaccin conforme à la revendication 14, dans laquelle le rapport de l'antigène à la protéine invasine recombinante purifiée est d'environ une partie d'antigène pour environ 0,0001 à environ 10 000 parties de protéine invasine purifiée.

16. Préparation de vaccin conforme à la revendication 14 ou à la revendication 15, dans laquelle la protéine invasine recombinante purifiée est pure à au moins 95%.

17. Préparation de vaccin conforme à n'importe laquelle des revendications 14 à 16, dans laquelle la protéine invasine recombinante purifiée est pure à au moins 97%.

18. Préparation de vaccin conforme à n'importe laquelle des revendications 14 à 17, dans laquelle la protéine invasine recombinante purifiée est dérivée d'un organisme pour lequel une immunité est souhaitée.

19. Préparation de vaccin conforme à n'importe laquelle des revendications 14 à 18, dans laquelle la protéine invasine recombinante purifiée comprend une séquence d'acides aminés dérivée d'une protéine invasine d'une bactérie choisie parmi *Shigella* spp., Salmonella spp. et E. coli entéroinvasif.

20. Préparation de vaccin conforme à la revendication 19, dans laquelle la protéine invasine recombinante purifiée est une protéine IpaC ou une protéine SipC.

21. Préparation de vaccin conforme à la revendication 19, dans laquelle la protéine invasine recombinante purifiée comprend une séquence d'acides aminés choisie à partir de la SEQ ID NO : 1 et la SEQ ID NO : 2.

22. Préparation de vaccin conforme à la revendication 19, dans laquelle la protéine invasine recombinante purifiée comprend un mutant choisi parmi
(i) une protéine pouvant être obtenue en éliminant 44% de la portion N terminale (environ 168 acides aminés) d'une IpaC par traitement avec une NdeI et une StuI (HisTag-B-C);
(ii) une protéine pouvant être obtenue en éliminant 39 % de la portion centrale (environ 150 acides aminés) d'une IpaC par traitement avec une StuI et une NsiI (HisTag-A-C);
(iii) une protéine pouvant être obtenue en éliminant 17% de la portion C terminale (environ 63 acides aminés) d'une IpaC par traitement avec une NsiI et une BamHI (HisTag-A-B);
(iv) une protéine constituée des acides aminés 62 à 363 d'une IpaC (IpaCΔ1);
(v) une protéine constituée des acides aminés -19 à 62 et 189 à 363 d'une IpaC (IpaCΔH);
(vi) une protéine constituée des acides aminés -19 à 176 et 261 à 363 d'une IpaC (IpaCΔII); et
(vii) une protéine constituée des acides aminés -19 à 260 d'une IpaC (IpaCΔIII).

23. Préparation de vaccin conforme à n'importe laquelle des revendications 14 à 22, dans laquelle la protéine invasine recombinante purifiée comprend une séquence d'acides aminés d'au moins 15 acides aminés.

24. Préparation de vaccin conforme à n'importe laquelle des revendications 14 à 23, dans laquelle la protéine invasine recombinante purifiée comprend une séquence d'acides aminés dans laquelle pas plus de 35 % des résidus d'acides aminés correspondant à des substitutions conservées.

25. Préparation de vaccin conforme à n'importe laquelle des revendications 14 à 24, dans laquelle l'antigène est conjugué chimiquement ou génétiquement fusionné à la protéine invasine recombinante.
